# EUROPEAN PATENT APPLICATION

(11) **EP 4 779 021 A1**
(43) Date of publication of application: **22.07.2026**
(21) Application number: 24865588.8
(22) Date of filing: 13.09.2024
(51) Int. Cl.: C12N 15/09, A01K 67/027, C12N 5/10, C12N 5/0735, C12N 15/85

(54) **MHC GENE CLUSTER-DEFICIENT ANIMAL**

(30) Priority: 13.09.2023 JP 2023148710
(71) Applicant: Tokyo Metropolitan Institute of Medical Science, Tokyo 156-8506 (JP)
(72) Inventor: SUZUKI Teruhiko, Tokyo 156-8506 (JP); HARA Takahiko, Tokyo 156-8506 (JP)
(74) Representative: J A Kemp LLP
(86) International application number: PCT/JP2024/080159
(87) International publication number: WO 2025/058090

(57) **Abstract**

A method for producing a cell that has a chromosome having the MHC genes (MHC genes 2) of a mammal 2 in the chromosome of a non-human mammal 1, and a chromosome in which all or a part of an MHC genes 1 is disrupted or deleted, wherein the production method comprises a step of disrupting or deleting all or a part of the MHC genes (MHC genes 1) derived from one non-human mammal (non-human mammal 1), from a cell heterozygously having a chromosome in which all or a part of the major histocompatibility complex (MHC) genes of one wild-type chromosome, between a pair of chromosomes of the non-human mammal, is substituted with the MHC genes of another mammal (mammal 2) other than the non-human mammal 1.

## Description

### [Technical Field]

The present invention relates to a chromosome in which MHC genes have been disrupted or deleted, and a cell having the aforementioned chromosome.

### [Background Art]

Major histocompatibility complex (MHC) is a membrane protein having the function of presenting intracellular antigens to T cells. MHC gene include: MHC class I, which presents intracellular antigens to CD8-positive T cells; MHC class II, which presents antigens incorporated from the outside to CD4-positive T cells; and non-classical MHC, which also contains molecules having physiological functions other than antigen presentation, such as regulation of antigen peptide presentation and regulation of NK cell activity. In mice, these MHC genes are present in the MHC region on chromosome 17. In the C57BL/6 strain, 39 types of MHC genes are present, although it is different depending on strain (Non Patent Literature 1).

When mice are used as animal models in pharmaceutical development, etc., utilizing T cell receptors (TCRs), it is necessary to allow individual mice that do not express mouse MHC to express human MHC, in order to reproduce human immune responses. However, since there are a large number of mouse MHC genes, it is extremely difficult to produce mice lacking all MHC genes, although genome editing technology is used, and thus, such mice have not yet been produced. For this reason, at present, mice lacking the classical MHC genes that play a main role in antigen presentation are utilized; or mice, in which the β2-macroglobulin (B2M) gene that forms a complex with MHC class I has been knocked out, are used as alternatives to MHC class I-deficient mice. However, in the former mice, non-classical MHC remains expressed, and in the latter mice, since the mouse MHC genes are not disrupted, human MHC needs to be expressed as a fusion protein with B2M. Thus, these are not ideal model systems.

### [CITATION LIST]

### [NON PATENT LITERATURE]

[Non Patent Literature 1] Shiina, T., Blancher, A., Inoko, H., and Kulski, J. K. (2017) Comparative genomics of the human, macaque and mouse major histocompatibility complex Immunology 150, 127-138 10.1111/imm.12624

### [Summary of Invention]

### [Technical Problem]

For these reasons, it has been desired to produce animals lacking MHC genes. If MHC genes-deficient mice could be produced, it would be possible to solve these problems. Moreover, if such MHC genes-deficient mice are used, it becomes possible to specifically analyze individual functions of the MHC proteins.

### [Solution to Problem]

As a result of intensive studies directed towards achieving the aforementioned objective, the present inventor has succeeded in generating cells having alleles in which mouse MHC genes are disrupted or deleted, thereby completing the present invention.

Specifically, the present invention is as follows.
[1] A chromosome of a non-human mammal, in which all or a part of the major histocompatibility complex (MHC) genes is deleted or disrupted.
[2] The chromosome according to the above [1], wherein the non-human mammal is a rodent.
[3] The chromosome according to the above [2], wherein the rodent is a mouse.
[4] A cell that homozygously or heterozygously comprises the chromosome according to the above [1].
[5] The cell according to the above [4], which is an embryonic stem cell.
[6] A non-human mammal having the chromosome according to the above [1].
[7] A method for producing a cell that has a chromosome having the MHC genes (MHC genes 2) of a mammal 2 in the chromosome of a non-human mammal 1, and a chromosome in which all or a part of an MHC genes 1 is disrupted or deleted, wherein the production method comprises a step of disrupting or deleting all or a part of the MHC genes (MHC genes 1) derived from one non-human mammal (non-human mammal 1), from a cell heterozygously having a chromosome in which all or a part of the major histocompatibility complex (MHC) genes of one wild-type chromosome, between a pair of chromosomes of the non-human mammal, is substituted with the MHC genes of another mammal (mammal 2) other than the non-human mammal 1.
[8] The method according to the above [7], wherein the cell is an embryonic stem cell.
[9] The method according to the above [7], wherein the step of disrupting or deleting the MHC genes 1 is carried out by genome editing technology using CRISPR/Cas9.
[10] The method according to the above [7], wherein the non-human mammal 1 is a rodent.
[11] The method according to the above [10], wherein the rodent is a mouse.
[12] The method according to the above [11], wherein the step of disrupting or deleting all or a part of the MHC genes 1 includes a step of disrupting or deleting, by genome editing technology using CRISPR/Cas9, all or a part of MHC genes consisting of a d1 genes, a d2 genes, a d3 genes, a d4 genes, a d5 genes and a d6 genes as described below, from a cell having a mouse MHC genes that consists of the d1 genes consisting of the genes H2-M10.2, H2-M10.1, H2-M10.3, H2-M10.4, H2-M11, H2-M9, H2-M1, H2-M10.5 and H2-M10.6, the d2 genes consisting of the genes H2-D1, H2-Q1, H2-Q2, H2-Q4, H2-Q5, H2-Q6, H2-Q7 and H2-Q10, the d3 genes consisting of the genes H2-T24, H2-T23, H2-T22, GM11127, H2-Bl, H2-T10, GM7030, GM8909 and H2-T3, the d4 genes consisting of the genes H2-Ob, H2-Ab1, H2-Aa, H2-Eb1 and H2-Eb2, the d5 genes consisting of the genes H2-DMa, H2-DMb2 and H2-DMb1, and the d6 genes consisting of the genes H2-K1, H2-Oa, H2-M5, H2-M3 and H2-M2.
[13] The method according to the above [12], wherein the genome editing vector used in the genome editing technology using CRISPR/Cas9 is a combination of vectors consisting of gRNAs having the nucleotide sequences as set forth in SED ID NOs: 53 to 67.
[14] A method for producing a non-human mammal 1 having a chromosome in which all or a part of the MHC genes 1 is disrupted or deleted, wherein the production method comprises a step of using the cell obtained by the method according to the above [7] to produce a non-human mammal 1 having a chromosome having the MHC genes 2 of the mammal 2 and a chromosome in which all or a part of the MHC genes 1 is disrupted or deleted, in a pair of chromosomes of the non-human mammal 1, and then using the non-human mammal 1 for breeding.
[15] A method for producing a cell having a chromosome in which all or a part of the MHC genes 1 is disrupted or deleted, wherein the production method comprises a step of recovering the cell from the non-human mammal 1 produced by the method according to the above [14].
[16] A method for producing a chromosome in which the MHC genes 1 is disrupted or deleted, wherein the production method comprises a step or recovering the chromosome from the non-human mammal 1 produced by the method according to the above [14], or the cell produced by the method according to the above [15].
[17] The genome editing vector used in the genome editing technology using CRISPR/Cas9 according to the above [13], wherein the genome editing vector includes a combination of vectors consisting of gRNAs having the nucleotide sequences as set forth in SED ID NOs: 53 to 67.

### [Advantageous Effects of Invention]

According to the present invention, an MHC genes-deficient animal is provided.

### [Brief Description of Drawings]

[Figure 1] Figure 1 is a schematic view showing recombination for substituting non-human MHC with human MHC in the present invention. The figure is a schematic view of a case where the non-human animal is a mouse.
[Figure 2] Figure 2 is a schematic view showing the MHC region in human chromosome.
[Figure 3] Figure 3 is a view showing a region that becomes a target when a genome editing targeting vector is used.
[Figure 4] Figure 4 is an outline of a method for producing an MHC region-humanized mouse.
[Figure 5] Figure 5 is a view showing the structures of targeting vectors. (A) Structure of TV3R-dHLA. (B) Structure of TV4-dH2. (C) Structure of TV-Tl1/2-BS. The positions of homology arms used as the targeting vectors on the genome are as shown in the figure.
[Figure 6] Figure 6 is a view showing isolation of A9-MRC5 fused cell clones having human chromosome 6.
   (A) Genomic DNA was prepared from the isolated A9-MRC5 fused cell clones (hChA9 #1, 5, 13, and 17), and whether the sequence on human chromosome 6 is present was analyzed by genomic PCR. (B) It was analyzed by FISH that human chromosome 6 is present in hChA9 #5. Red: human chromosome 6 centromere-specific probe.

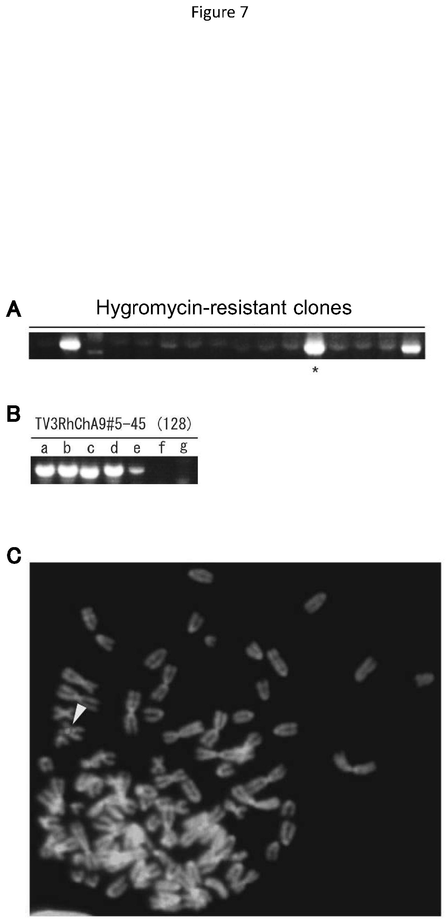

[Figure 7] Figure 7 is a view showing the targeting of TV3R.
   (A) TV3R-dHLA targeting was performed on an A9-MRC5 fused cell clone, and hygromycin-resistant clones were isolated and were analyzed by genomic PCR for the occurrence of recombinant introduction. * indicates the TV3RhChA9 #5-45 (128) clone. (B) TV3RhChA9 #5-45 (128) was recloned and was analyzed by genomic PCR. (C) TV3RhChA9 #5-45a was analyzed by FISH. Red: human chromosome 6 centromere-specific probe.

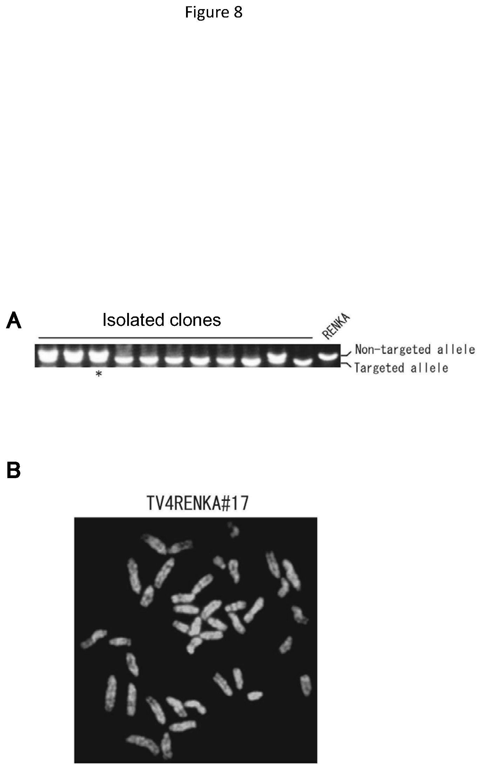

[Figure 8] Figure 8 is a view showing the targeting of TV4.
   (A) The targeting of TV4-dH2 was analyzed by genomic PCR. * indicates the TV4RENKA #17 clone. (B) It was confirmed by DAPI staining that the number of chromosomes in TV4RENKA #17 is 40.

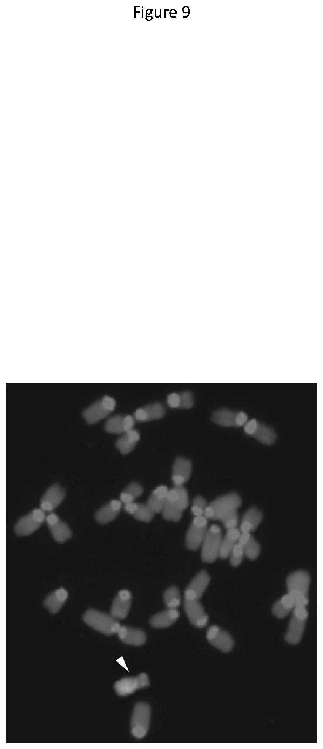

[Figure 9] Figure 9 is a view showing isolation of the TV3-4RENKA clone. FISH analysis was performed on TV3-4RENKA #17/5-45a-3 cells introduced with modified human chromosome 6. The arrowhead indicates the introduced modified human chromosome 6. The number of chromosomes was confirmed to be 41. Red: Mouse Cot-1 (probe for mouse genomic sequence), Purple: RP11-54H13 (probe within the human MHC region).

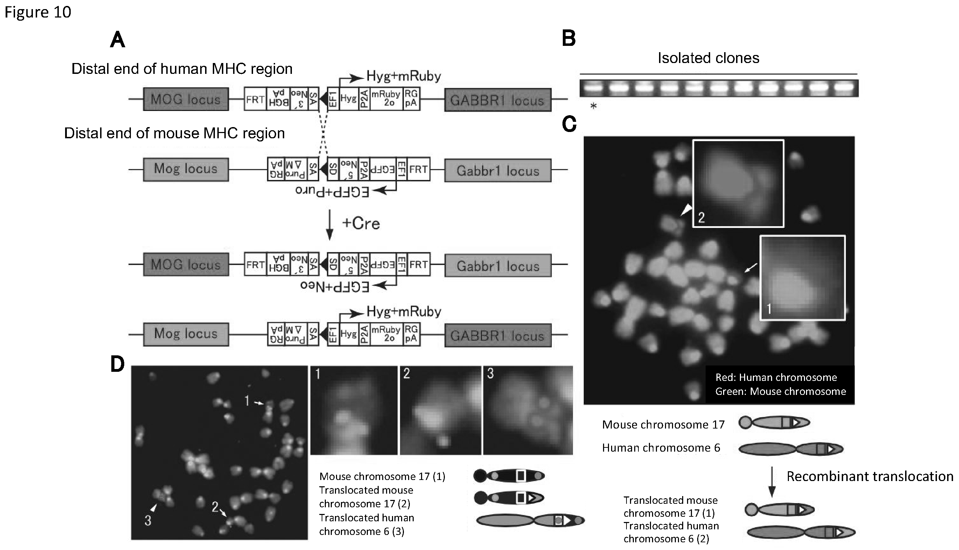

[Figure 10] Figure 10 is a view showing isolation of TV3-4RENKATl3/4 clones. (A) Recombinant translocation by Cre/loxP. (B) Confirmation of translocation by genomic PCR. * indicates TV3-4RENKATl3/4 #17-1. (C) TV3-4RENKATl3/4 #17-1 was analyzed by FISH. The numbers in the FISH images correspond to the numbers of the translocated chromosomes in the schematic view. Red: human Cot-1 (probe for human genomic sequence), Green: mouse Cot-1 (probe for mouse genomic sequence) (D) TV3-4RENKATl3/4 #17-1 was analyzed by FISH. The numbers in the FISH images correspond to the numbers of the translocated chromosomes in the schematic view. Red: RP11-54H13 (probe within the human MHC region), Green: RP23-147G23 (probe on the centromere side of mouse chromosome 17 MHC region), Purple: RP23-119G24 (probe on the telomere side of mouse chromosome 17 MHC region).

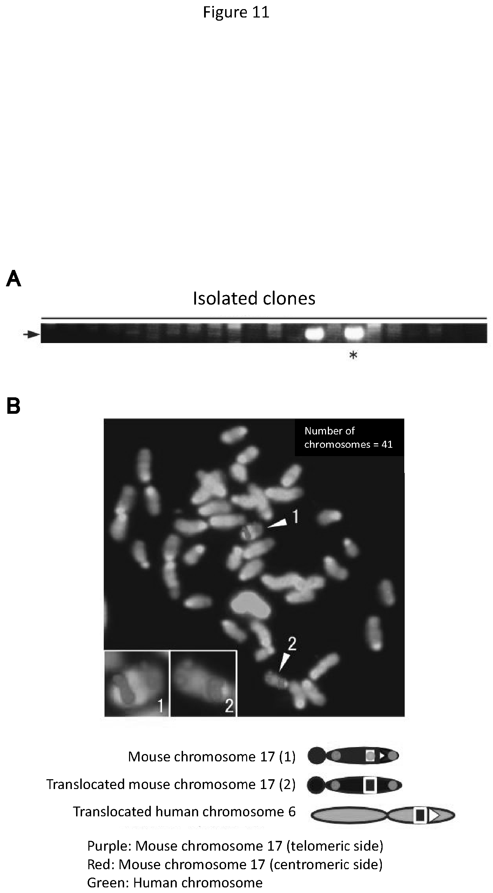

[Figure 11] Figure 11 is a view showing isolation of TV3-4RENKATlb clones. (A) Chromosomal translocation was analyzed by genomic PCR. * indicates TV3-4RENKATlb #38 clone. (B) TV3-4RENKATlb #38 was analyzed by FISH. The numbers in the FISH images correspond to the numbers of the translocated chromosomes in the schematic view. Red: RP23-147G23 (probe on the centromere side of mouse chromosome 17 MHC region), Green: human Cot-1(probe for human genomic sequence), Purple: RP23-306H20 (probe on the telomere side of mouse chromosome 17 MHC region).

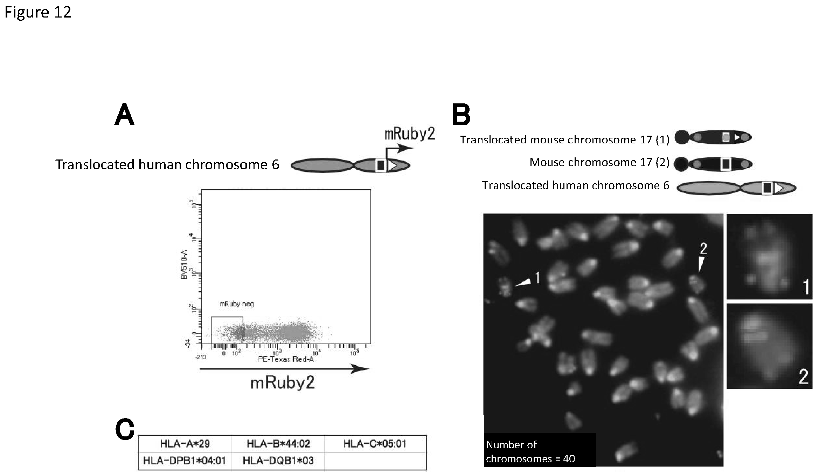

[Figure 12] Figure 12 is a view showing isolation of TV3-4RENKATlbdh6 clones. (A) Sorting of TV3-4RENKATlbdh6. (B) TV3-4RENKATlb #38dh6 #3 was analyzed by FISH. The numbers in the FISH images correspond to the numbers of the translocated chromosomes in the schematic view. The number of chromosomes was confirmed to be 40. Red: RP23-147G23 (probe on the centromere side of mouse chromosome 17 MHC region), Green: human Cot-1 (probe for human genomic sequence), Purple: RP23-361C19 (probe on the telomere side of mouse chromosome 17 MHC region). (C) The results obtained by analyzing the HLA haplotype of TV3-4RENKATlbdh6 #3 by whole genome sequencing.

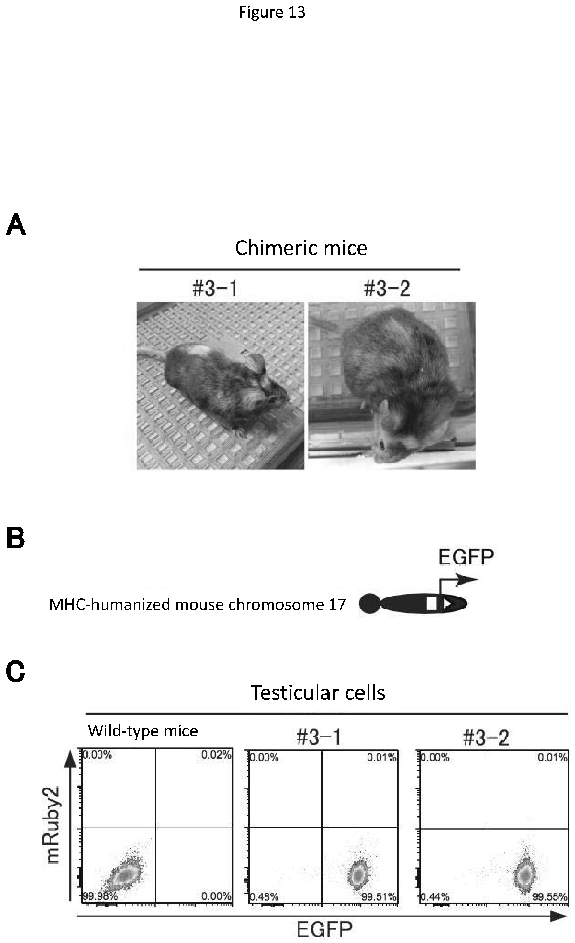

[Figure 13] Figure 13 is a view showing MHC-humanized chimeric mice.
   (A) The appearance of chimeric mice derived from TV3-4RENKATlbdh6 #3. (B) The structure of MHC-humanized mouse chromosome 17. The EGFP expression cassette was inserted into the distal side of the MHC region. (C) The expression of EGFP in the testicular cells of chimeric mice derived from TV3-4RENKATlbdh6 #3 was analyzed by flow cytometry.

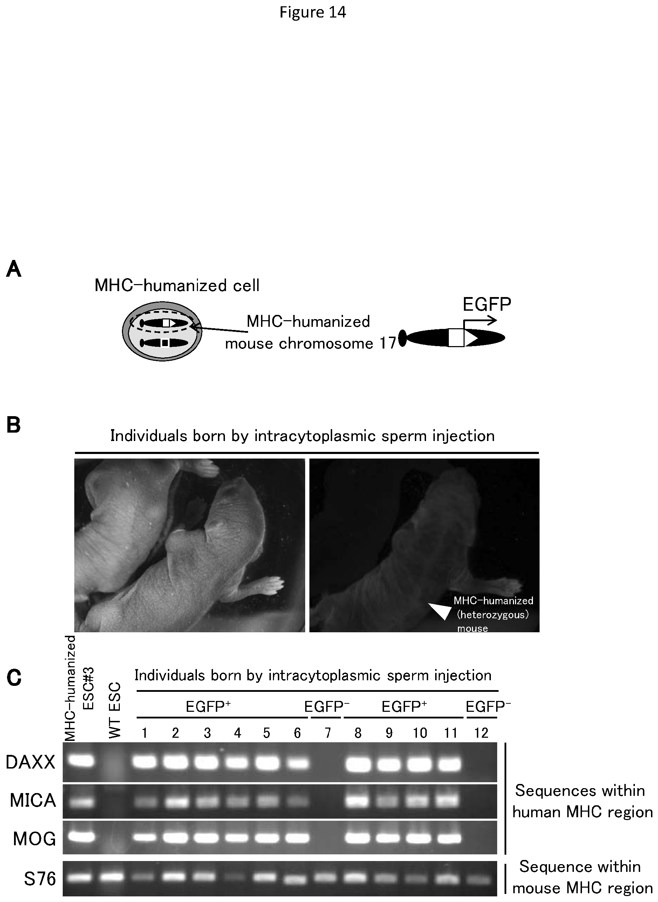

[Figure 14] Figure 14 is a view showing generation of MHC-humanized (heterozygous) mice.
   (A) MHC-humanized (heterozygous) cells have MHC-humanized mouse chromosome 17, in which an EGFP expression cassette was inserted into the distal side of the MHC region. (B) Individuals born by intracytoplasmic sperm injection using the testicular cells of chimeric mice derived from TV3-4RENKATlbdh6 #3. Left: bright-field image. Right: EGFP fluorescence detected. (C) Genomes were prepared from offsprings obtained by intracytoplasmic sperm injection and were analyzed by PCR for the presence of sequences within the human MHC region. Individual Nos. 7 and 12 were EGFP-negative offsprings.

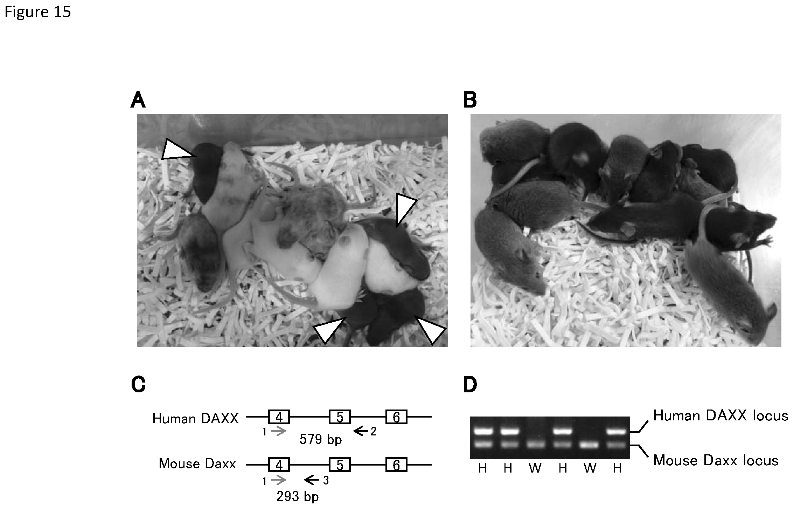

[Figure 15] Figure 15 is a view showing generation of MHC-humanized (heterozygous) mice from XO ES cells.
   (A) Chimeric mice generated from TV3-4XOTlbdh6 #11-1-4-57-3 clones. Arrowhead: 100% chimeric mouse. (B) Offsprings of an XO chimeric mouse (female) and a C57BL/6 (male). Black-haired individuals are not born unless XO ES cells are transmitted through the germline. (C) Genome typing method targeting the DAXX locus within the MHC region. Three primers, 1 to 3, are used to determine whether the MHC region is derived from human or mouse. The red arrows indicate the positions of primers designed at portions in which the sequences are matched between human and mouse. Black arrows indicate human- or mouse-specific primers. (D) Example of genotyping of offsprings obtained by breeding an XO chimeric mouse (female) with a C57BL/6 (male). W: wild-type mouse; H: MHC-humanized (heterozygous) mouse.

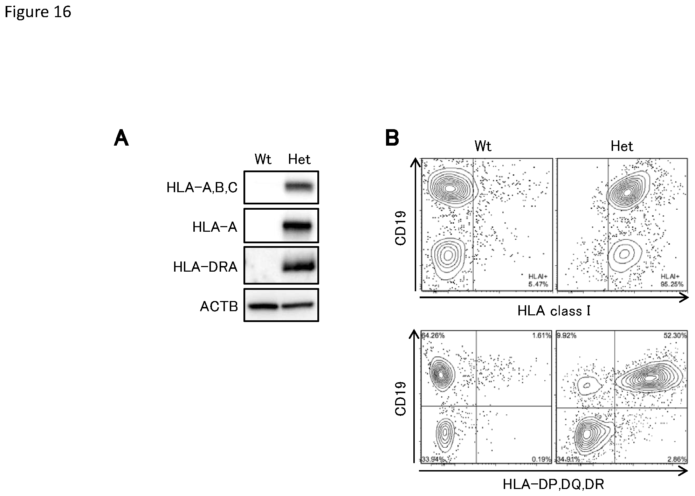

[Figure 16] Figure 16 is a view showing the results of human MHC protein expression analysis in MHC-humanized (heterozygous) mice.
   (A) Human MHC protein expression in splenocytes was analyzed by Western blotting. Wt: wild-type mouse-derived splenocytes; Het: MHC-humanized (heterozygous) mouse-derived splenocytes. (B) Surface expression of human MHC protein in splenocytes was analyzed by flow cytometry. Wt: wild-type mouse-derived splenocytes; Het: MHC-humanized (heterozygous) mouse-derived splenocytes.

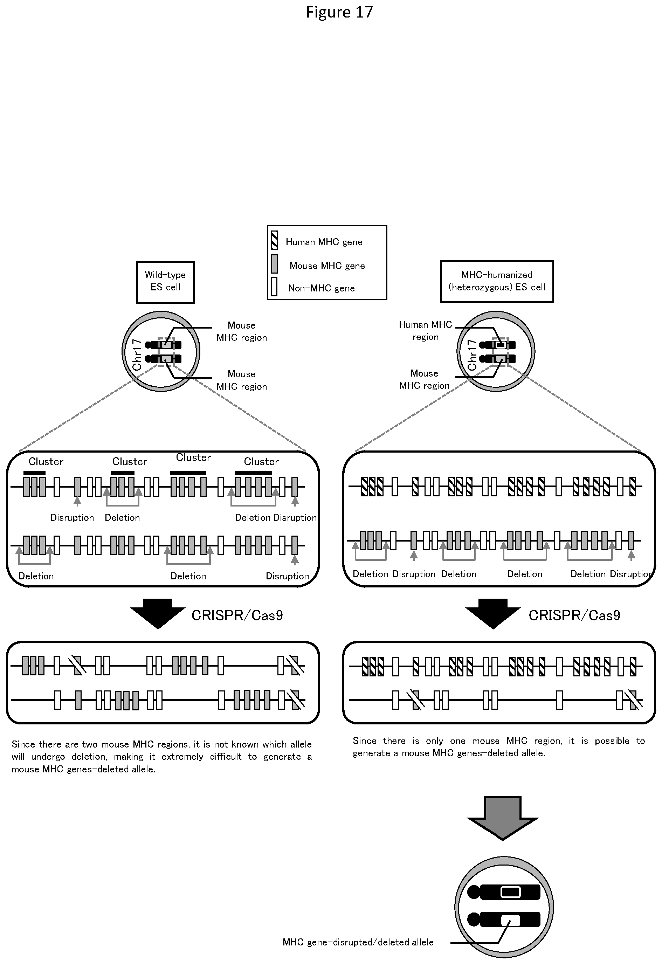

[Figure 17] Figure 17 is a schematic view showing generation of mouse MHC genes-deficient alleles using MHC-humanized (heterozygous) ES cells.

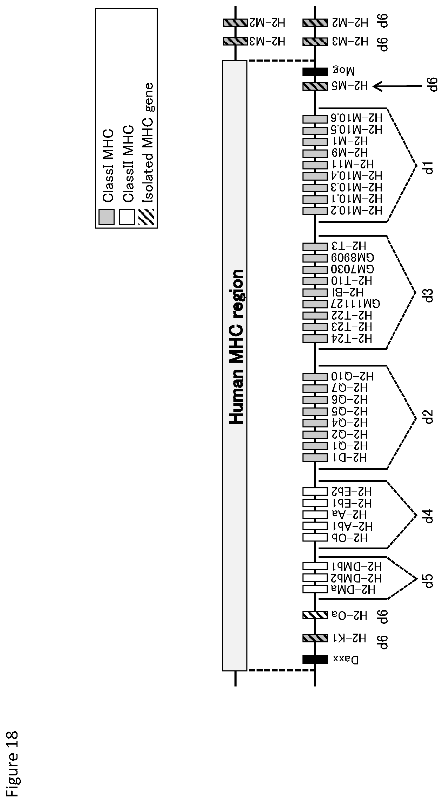

[Figure 18] Figure 18 is a view showing the structure of the MHC region in MHC-humanized (heterozygous) ES cells and distribution of the mouse MHC genes. The upper row shows MHC-humanized alleles, and the lower row shows wild-type alleles. Mouse MHC genes were classified into six gene groups ranging from d1 to d6.

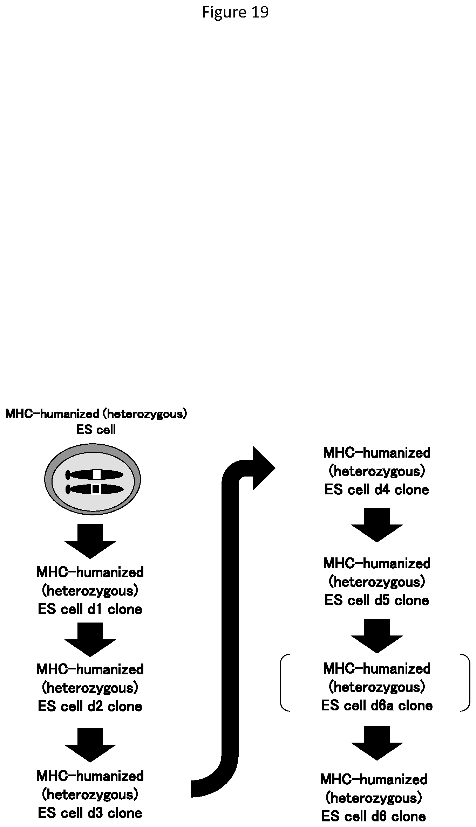

[Figure 19] Figure 19 is a view showing the procedures for generating mouse MHC-deficient alleles.

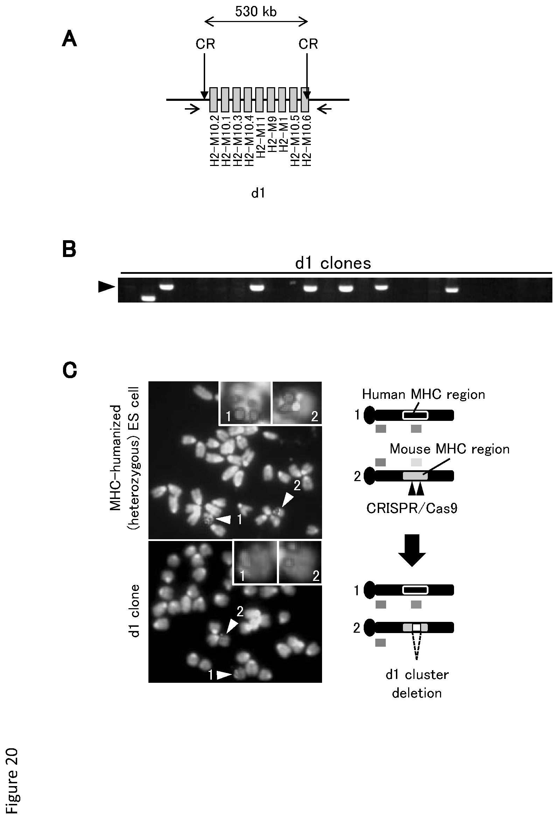

[Figure 20] Figure 20 is a view showing isolation of d1 clones using CRISPR/Cas9. (A) A schematic view of the d1 cluster. CR indicates the target site of CRISPR/Cas9. The arrows indicate the positions of the primers used to confirm deficiency. (B) The isolated clones were analyzed by genomic PCR. The arrowhead indicates the expected position of the PCR amplified product. (C) FISH analysis of clones that were positive for genomic PCR. Red indicates mouse chromosome 17, purple indicates the human MHC region, and green indicates the staining image using a probe for the d1 cluster.

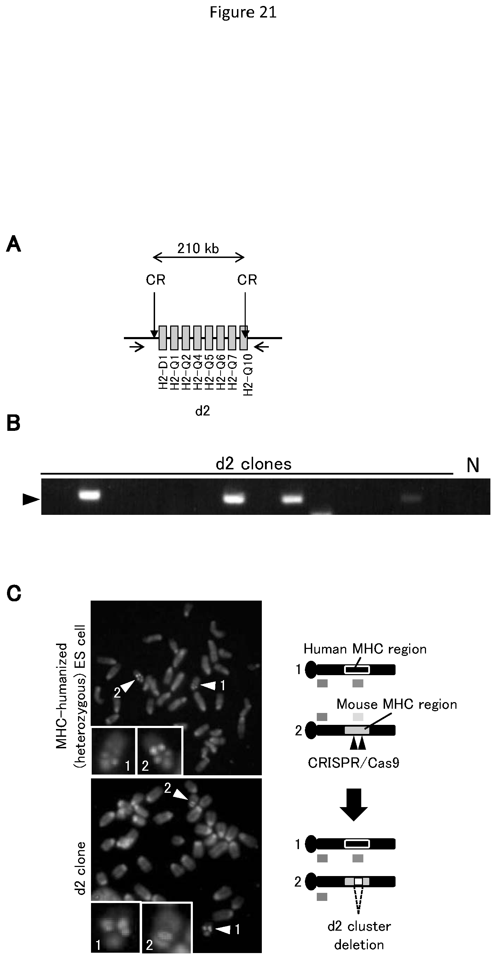

[Figure 21] Figure 21 is a view showing isolation of d2 clones using CRISPR/Cas9. (A) A schematic view of the d2 cluster. CR indicates the target site of CRISPR/Cas9. The arrows indicate the positions of the primers used to confirm deficiency. (B) The isolated clones were analyzed by genomic PCR. The arrowhead indicates the expected position of the PCR amplified product. (C) FISH analysis of clones that were positive for genomic PCR. Red indicates mouse chromosome 17, purple indicates the human MHC region, and green indicates the staining image using a probe for the d2 cluster.

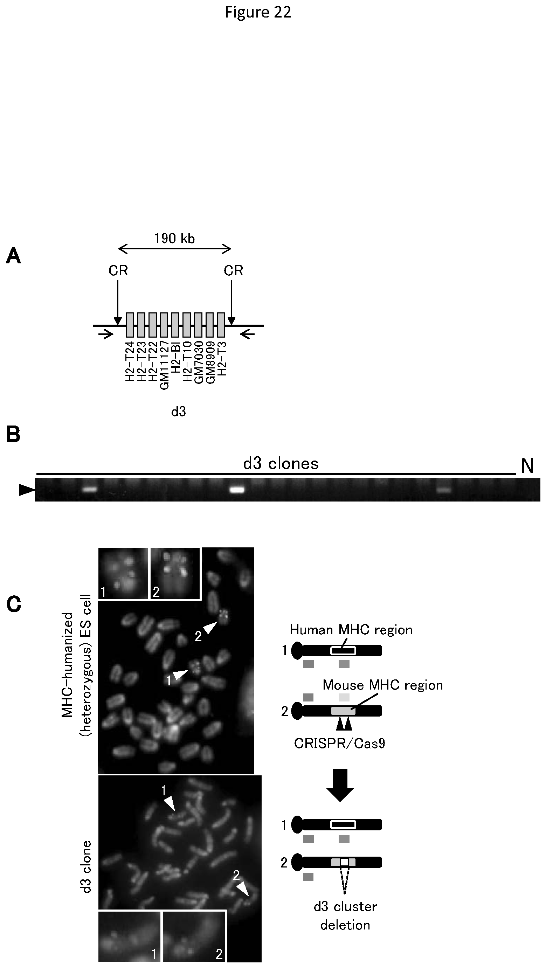

[Figure 22] Figure 22 is a view showing isolation of d3 clones using CRISPR/Cas9. (A) A schematic view of the d3 cluster. CR indicates the target site of CRISPR/Cas9. The arrows indicate the positions of the primers used to confirm deficiency. (B) The isolated clones were analyzed by genomic PCR. The arrowhead indicates the expected position of the PCR amplified product. (C) FISH analysis of clones that were positive for genomic PCR. Red indicates mouse chromosome 17, purple indicates the human MHC region, and green indicates the staining image using a probe for the d3 cluster.

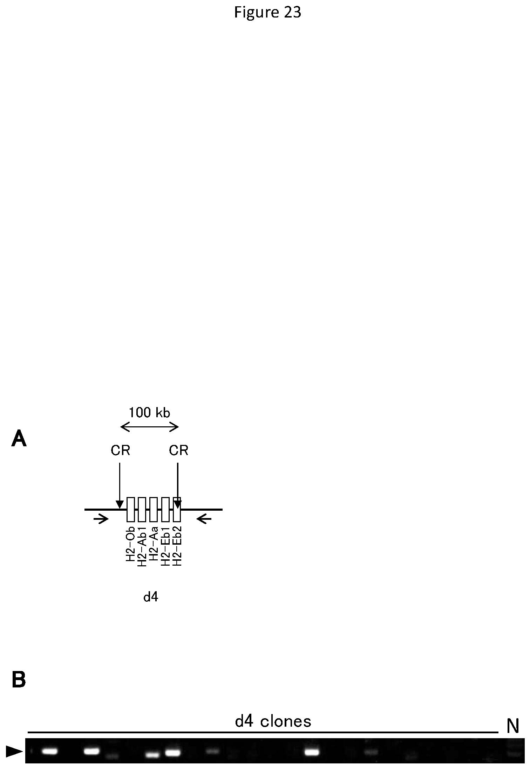

[Figure 23] Figure 23 is a view showing isolation of d4 clones using CRISPR/Cas9. (A) A schematic view of the d4 cluster. CR indicates the target site of CRISPR/Cas9. The arrows indicate the positions of the primers used to confirm deficiency. (B) The isolated clones were analyzed by genomic PCR. The arrowhead indicates the expected position of the PCR amplified product.

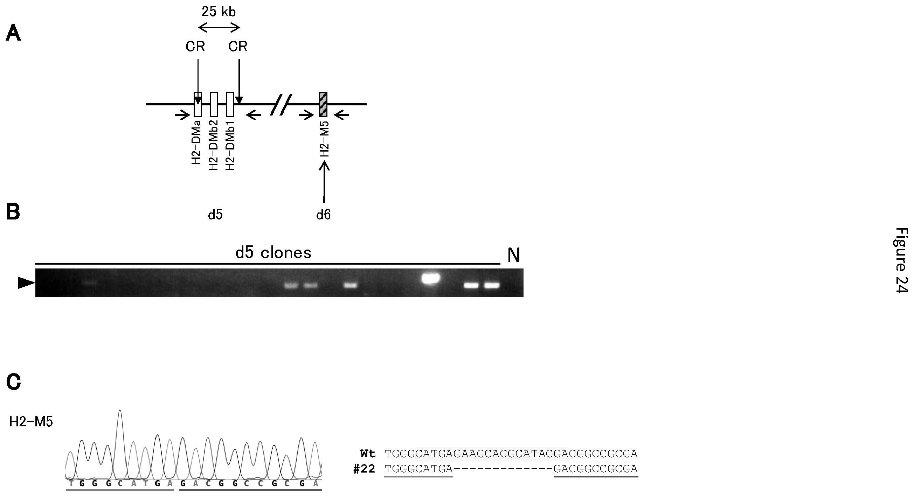

[Figure 24] Figure 24 is a view showing isolation of d5 clones using CRISPR/Cas9. (A) A schematic view of the d5 cluster. CR indicates the target site of CRISPR/Cas9. The arrows indicate the positions of the primers used to confirm deficiency. (B) The isolated clones were analyzed by genomic PCR. The arrowhead indicates the expected position of the PCR amplified product. (C) The H2-M5 gene disruption in genomic PCR-positive clones was analyzed by sequence analysis.

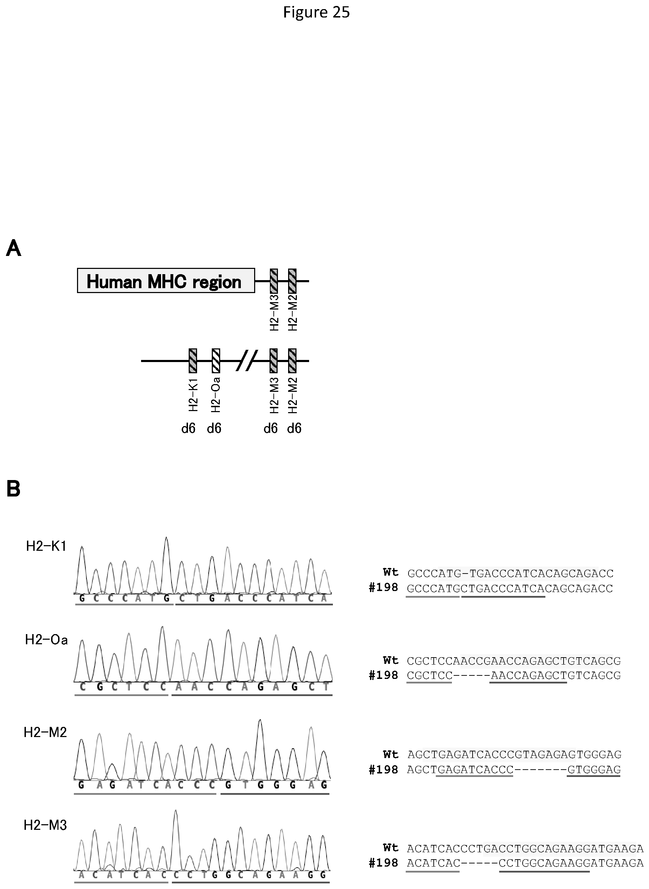

[Figure 25] Figure 25 is a view showing isolation of d6 clones using CRISPR/Cas9. (A) A schematic view of the d6 genes. (B) The results of the sequence analysis of the isolated d6 clone #198 are shown.

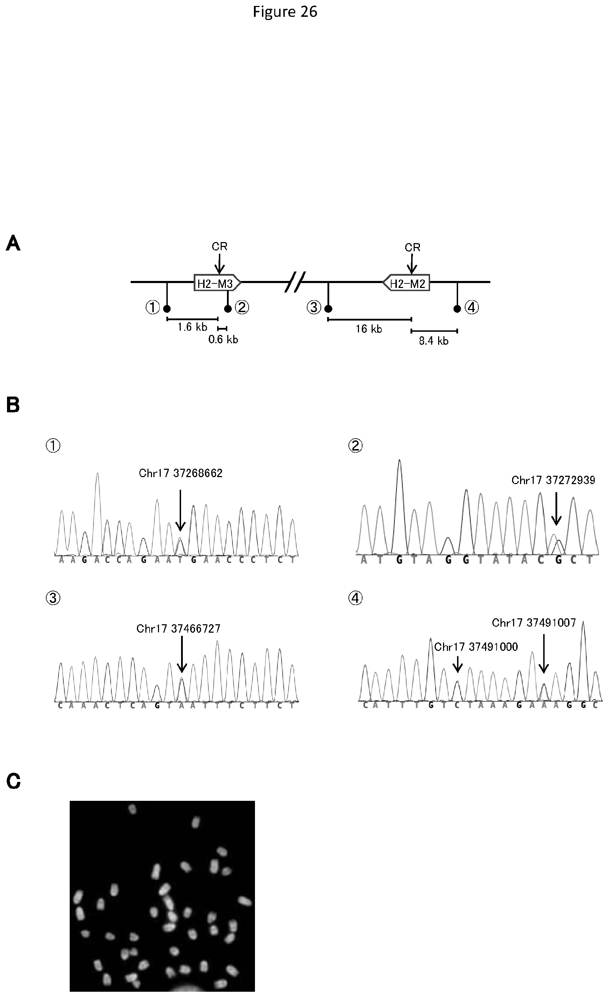

[Figure 26] Figure 26 is a view showing SNP analysis near H2-M2.
   (A) The positions of SNPs near H2-M2 are indicated by black circles. (B) Regarding the d6 clone #198, SNPs 1 and 2 (indicated by the numbers in the circles) located upstream or downstream of H2-M2 were analyzed. (C) The number of chromosomes of the d6 clone #198 was analyzed.

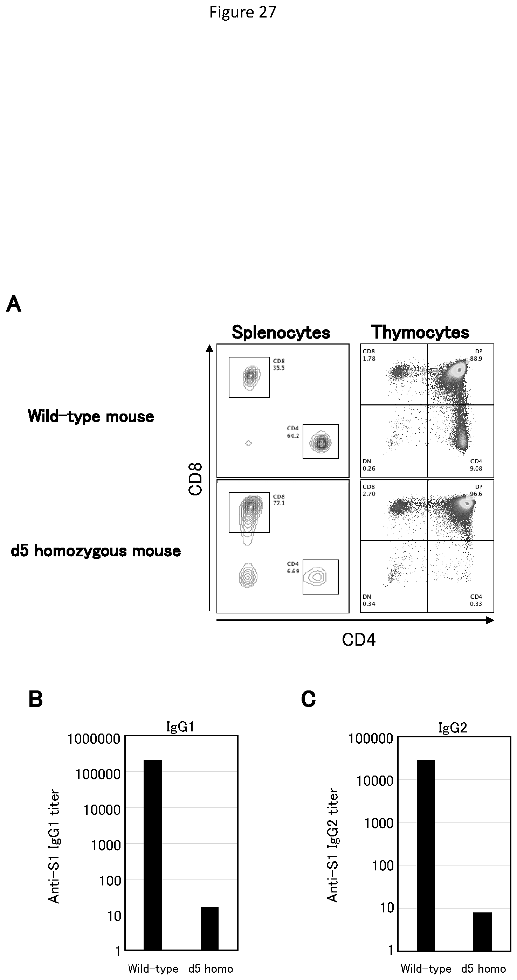

[Figure 27] Figure 27 shows T cell differentiation in d5 homozygous mice.
   (A) Splenocytes or thymocytes were isolated from wild-type or d5 homozygous mice, and CD4 or CD8 expression in CD3-positive cells was analyzed. (B, C) Wild-type or d5 homozygous mice were immunized with SARS-CoV-2 S protein, and anti-S1 IgG1 (B) or IgG2 (C) antibodies in the serum were analyzed by ELISA.

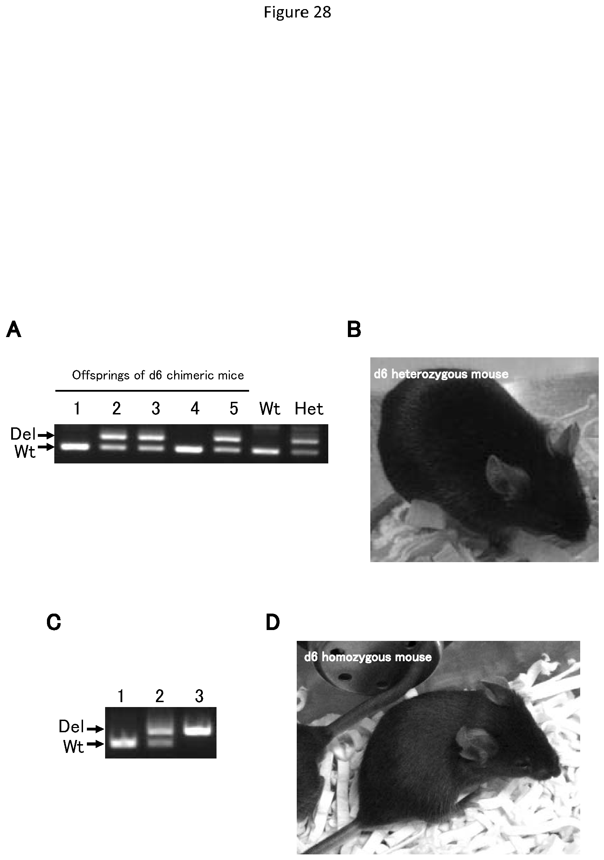

[Figure 28] Figure 28 shows production of mouse MHC genes-deficient mice.
   (A) The results obtained by analyzing offspring obtained by breeding a chimeric mouse produced from a d6ES clone with a wild-type mouse according to genomic PCR. (B) Germline-transmitted d6 heterozygous mice. (C) The results obtained by analyzing offspring obtained by breeding a d6 heterozygous male mouse with a d6 heterozygous female mouse according to genomic PCR. (D) A d6 Homozygous mouse having Individual number 3 obtained by breeding.

### [Description of Embodiments]

The present invention relates to a chromosome of a non-human mammal, in which the major histocompatibility complex (MHC) genes are deleted or disrupted. In addition, the present invention relates to a cell that homozygously or heterozygously comprises the aforementioned chromosome, and further, to a non-human mammal, etc. produced from the aforementioned cell, the non-human mammal, etc. having the chromosome in which the MHC genes are deleted or disrupted.

Furthermore, the present invention provides a method for producing, from a cell heterozygously having a chromosome in which the MHC genes (also referred to as "MHC genes 1") of one wild-type chromosome, between a pair of chromosomes of one non-human mammal (referred to as "non-human mammal 1"), is substituted with the MHC genes (also referred to as "MHC genes 2") of another mammal other than the non-human mammal 1, a cell having a chromosome having the MHC genes 2 of the mammal 2 in the chromosome of the non-human mammal 1, and a chromosome in which all or a part of the MHC genes 1 of the non-human mammal 1. The present production method comprises a step of disrupting or deleting the MHC genes 1 in the other wild-type chromosome of the non-human mammal 1.

### 1. Outline

Since there is one pair of sequences on the autosome, gene disruption or the deletion of a genomic sequence, using genome editing technology, randomly occurs in one or both alleles. However, in order to generate an MHC-deficient non-human mammal, all MHC genes must be disrupted or deleted in one allele. If the MHC region of a non-human mammal is present in only one allele, it is possible to produce an MHC-deficient allele. Thus, using a mouse as an example, the present inventor has generated MHC-humanized (heterozygous) ES cells in which the mouse MHC region is present in only one allele. Using these ES cells, the present inventor has devised a method for generating a mouse MHC-deficient allele by disrupting or deleting the mouse MHC gene on the same allele, thereby establishing ES cells having a mouse MHC-deficient allele.

Hereinafter, generation of a cell heterozygously having a chromosome in which the MHC genes 1 of one wild-type chromosome, between a pair of chromosomes of one wild-type non-human mammal, is substituted with the MHC genes 2 of another mammal (referred to as "mammal 2") other than the non-human mammal 1, will be explained. Next, a method for disrupting or deleting all or a part of the MHC genes 1 in the other wild-type chromosome of the non-human mammal 1, from the chromosome of the cell heterozygously having the chromosome, will be explained.

Non-human mammal 1 is an animal whose own MHC region is substituted with the MHC region of another mammal. The type of the non-human mammal 1 is not particularly limited, and examples of the non-human mammal 1 may include rodents such as mice, rats, guinea pigs, rabbits, and hamsters; livestock animals such as bovines, horses, pigs, and sheep; pet animals such as dogs and cats; and primates such as Japanese macaques and common marmosets. The non-human mammal 1 is not limited to the above-mentioned animals.

Mammal 2 is an animal whose MHC region is to be substituted with that of the non-human mammal 1. The type of the mammal 2 is not particularly limited, and examples of the mammal 2 may include the above-described non-human mammals 1 as well as humans.

2. Production of cells in which MHC of non-human mammal 1 has been substituted with MHC of mammal 2, and non-human mammal (1) Method for substituting MHC region of non-human mammal 1 with MHC region of mammal 2

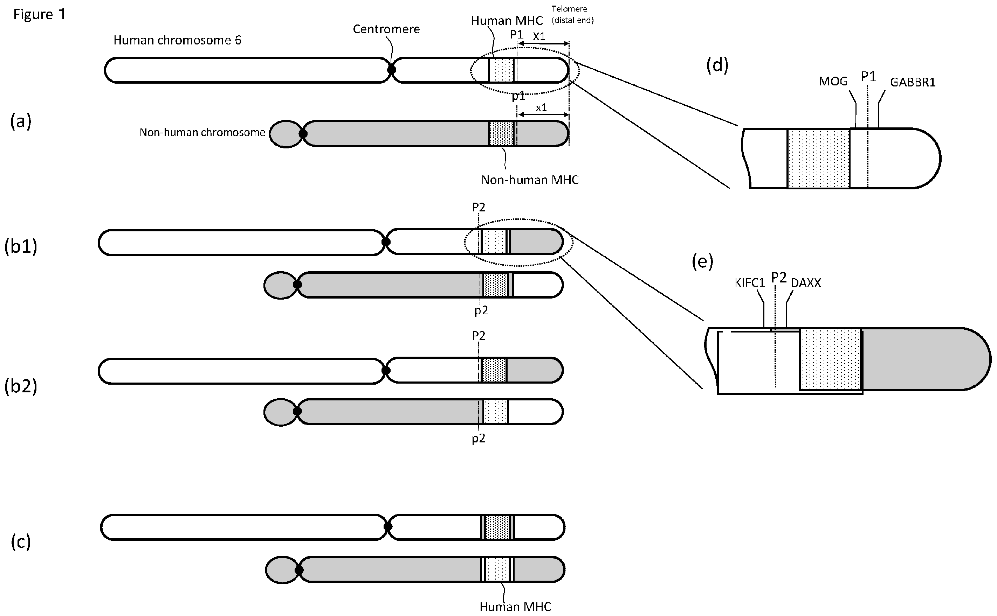

Figure 1 shows an overview of the substitution of the MHC region of the non-human mammal 1 with the MHC region of the mammal 2. In Figure 1, for convenience of explanation, the mammal 2 is explained using a human as an example.

In Figure 1, (a) is a schematic view of the chromosome 6 of mammal 2 (a human) and the chromosome of the non-human mammal 1 (a non-human) before substitution, and (c) is a schematic view of the chromosome after substitution of the MHC region. The substitution of the MHC region is carried out by utilizing two-step recombination: a translocation recombination from (a) to (b1) and a translocation recombination from (b1) to (c) (referred to as "two-step recombination 1"), or alternatively, by utilizing two-step recombination: a translocation recombination from (a) to (b2) and a translocation recombination from (b2) to (c) (referred to as "two-step recombination 2"). In the present invention, the order of recombination is arbitrary, and either the two-step recombination 1 or the two-step recombination 2 may be used.

In Figure 1, the telomere side seen from the MHC region targeted for translocation recombination (in the present description, also referred to as "substitution" or simply "recombination") is referred to as a distal side, and the centromere side is referred to as the proximal side. The distal side to the human MHC region, namely, an arbitrary position between the human MHC region and the distal end, which serves as a boundary of recombination, is designated as P1, and an arbitrary position distal to the non-human MHC region, namely, between the non-human MHC region and the distal end, which serves as a boundary of recombination and corresponds to the P1, is designated as p1. In addition, in Figure 1, the proximal side of the human MHC region as a target of recombination, namely, an arbitrary position between the human MHC region and the centromere, which serves as a boundary of recombination, is designated as P2, and the proximal side of the non-human MHC region, namely, an arbitrary position between the non-human MHC region and the centromere, which serves as a boundary of recombination and corresponds to P2, is designated as p2.

In this context, in the two-step recombination 1, the recombination from (a) to (b1) is a first recombination, which involves the recombination of the region from P1 to the distal end (telomere) with the region from p1 to the distal end (telomere). The recombination from (b1) to (c) is a second recombination, which involves the recombination of the region from P2 to the distal end (telomere) with the region from p2 to the distal end (telomere).

On the other hand, in the two-step recombination 2, the recombination of the region from the centromeric side (P2) to the distal end of the human MHC region, which is the target of recombination in Figure 1, with the region from the centromeric side (p2) to the distal end of the non-human MHC region occurs as a "first recombination" (Figure 1(b2)). In this step, the recombination of the region from P1 to the distal end of the human chromosome with the region from p1 to the distal end of the non-human chromosome has not occurred. Thereafter, the recombination of the region from P1 to the distal end of the human chromosome with the region from p1 to the distal end of the non-human chromosome occurs as a "second recombination."

After recombination has been completed, MHC-humanized cells, for example, ES cells, can be produced and can be then transplanted into early embryos of non-human mammals 1 to produce chimeric non-human animals. After recombination has been completed, it is preferable to remove any human chromosomes remaining in the cells. Human chromosomes can be shed and removed by subculturing the cells in the absence of a selective drug. Moreover, while chromosomes in non-human mammals are diploids, among the chromosomes of non-human mammals after completion of the recombination in the present invention, the aspects of chromosomes, in which the MHC region has been substituted with the MHC region of a human chromosome, may be either homozygous (the MHC regions of both of the two chromosomes have been humanized) or heterozygous (the MHC region of one chromosome has been humanized). However, chromosomes are preferably heterozygous in order to delete or disrupt the MHC region in the after-mentioned non-human chromosome.

Therefore, subsequently, MHC-humanized heterozygous animals can be produced by natural breeding or intracytoplasmic sperm injection, etc. It is to be noted that MHC-humanized homozygous animals can be further produced by natural breeding or intracytoplasmic sperm injection, etc.

Herein, human MHC is the same molecule as HLA, and the genes that determine this molecule are arranged in tandem on chromosome 6. The MHC region of non-human mammal 1 is different depending on the animal. For example, it is located on chromosome 17 in mice, chromosome 20 in rats, chromosome 12 in rabbits, and chromosome 7 in pigs. The non-human mammal 1 is not limited to the aforementioned animals, and other examples thereof may include chromosome 20 in horses, chromosome 23 in bovines, chromosome 12 in dogs, and chromosome 4 in common marmosets.

It is to be noted that when the genes encoding MHC in a genetic region are described, the term "MHC region" is sometimes used. Moreover, among the non-human mammals 1, since the MHC region of, for example, a mouse is located on the long arm, when a mouse chromosome is used as a non-human chromosome in Figure 1 for example, the orientation of the mouse long arm and short arm is opposite to that of the human long arm and short arm.

The MHC region as a target to be recombined may be the entire MHC region or a part thereof. In class I, it may be either a classical class I molecule or a non-classical class I molecule. In humans, there are three types of classical class I molecules, namely, HLA-A, HLA-B, and HLA-C; and non-classical class I molecules include HLA-E, HLA-F, HLA-G, MICA, and MICB.

Human class II genes are located in the human MHC class II region, and the DR, DQ, and DP loci encode the major products of this region. It is to be noted that mouse MHC also contains mouse H2-M2 and H2-M3, but that these MHCs do not need to be included in the target region for substitution in the present invention. The class III region contains various genes other than MHC, and in humans, it is the region located distal to HLA-DRA and proximal to MICB.

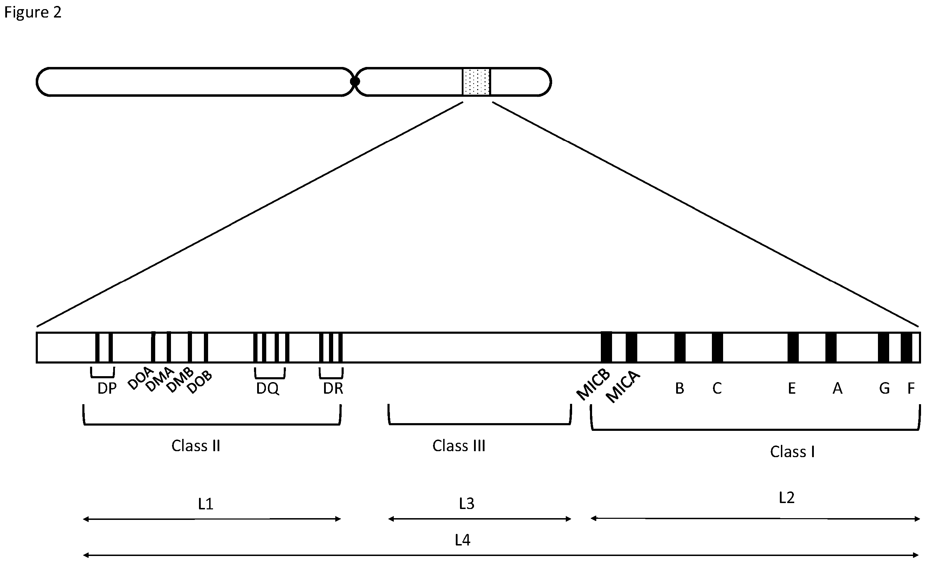

For example, Figure 2 is a schematic view showing human MHC regions. The MHC region as a target to be substituted may be only the class I region, only the class II region, or only the class III region, or a combination of these regions (including all combinations). Furthermore, the class I region may be a region containing classical class I MHC genes, a region containing non-classical class I MHC genes, or both of these regions. Therefore, in the present invention, the region constituting each class may be, for example, a region of any one of HLA-A, HLA-B, HLA-C, HLA-E, HLA-F, HLA-G, MICA and MICB, or a combination of two or more of these (including all combinations), for class I; and a region of any one of HLA-DP, HLA-DQ, HLA-DR, HLA-DO and HLA-DM, or a combination of two or more of these (including all combinations), for class II. The class III region contains various genes other than MHC, and in humans, it is the region located distal to HLA-DRA and proximal to MICB.

Herein, as described above, the MHC region as a target to be recombined may be the entire region or a part of the region. Therefore, depending on the selection of the MHC region as a target, the above-described P1, p1, P2, and p2 may also be located in an unselected MHC region in some cases.

In the present invention, it is preferable that the human MHC class I region includes the entire region from MICB to HLA-F (the region indicated by L2 in Figure 2), and that the class II region includes the entire region from HLA-DP to HLA-DR (the region indicated by L1 in Figure 2). In addition, the class III region is the region located distal to HLA-DRA and proximal to MICB (the region indicated by L3 in Figure 2). In one aspect of the present invention, it is more preferable that the entire MHC region shown in Figure 2 (the region indicated by L4 in Figure 2) is substituted.

### (2) Targeting vector

(2-1) First recombination in two-step recombination 1 and second recombination in two-step recombination 2

In the present invention, the vector used to induce recombination is referred to as a targeting vector. The targeting vector used herein is not limited, as long as it induces the recombination described above. It may be either a vector that utilizes a recombinase recognition sequence and a recombinase, or a vector that utilizes a genome editing tool. As necessary, a genome editing tool can also be utilized as a tool for cleaving the chromosome at the positions of P1 and p1 described above. Since the chromosome can be cleaved at a position of interest if a genome editing tool is utilized, it is possible to facilitate the subsequent recombination.

### < Recombination utilizing recombinase >

In recombination utilizing a recombinase, for example, a recombinase recognition sequence called loxP and a recombinase called Cre are utilized. The Cre/loxP specific recombination system is well known. The Cre enzyme is a 343-amino acid protein derived from the P1 phage, and it recognizes a specific 34-bp nucleotide sequence called the loxP site and is capable of performing site-specific recombination. The loxP site can be divided into three parts, namely, 13 nucleotides, 8 nucleotides, and 13 nucleotides. The sequence consisting of 13 nucleotides has a complementary inverted repeat structure. Meanwhile, the sequence consisting of 8 nucleotides has a role of determining the orientation of the loxP site. Many mutant loxPs, called Lox511 and lox2272, are also known, and these recombinase recognition sequences can also be utilized.

In another aspect of the present invention, in addition to the Cre/loxP system, other examples of the site-specific recombination systems using a site-specific recombinase that can be used herein may include the Flp/FRT system derived from yeast plasmid 2µ, the PhiC31 integrase system derived from bacteriophage PhiC31, and the Bxb1 integrase system derived from bacteriophage Bxb1.

In Figure 1, the targeting vector for recombination from (a) to (b1) or recombination from (b2) to (c) is a combination of the following targeting vector 1 and targeting vector 2, or is the following targeting vector 3. The combination of the targeting vector 1 and the targeting vector 2 is a vector that induces recombination utilizing a recombinase recognition sequence and a recombinase, while the targeting vector 3 is a vector that induces recombination utilizing a genome editing tool.

### (i) Targeting vector 1

Targeting vector 1 is incorporated into arbitrary position P1 on the distal side of the MHC (human MHC) region, wherein the MHC region is located on the short arm of human chromosome 6 and becomes a target of recombination. The targeting vector 1 induces translocation recombination between the region from above-described P1 to the distal end, and the region from position p1 that is on the distal side of a non-human MHC region as a target of recombination and corresponds to the above-described P1 to the distal end in a non-human mammalian chromosome. The targeting vector 1 comprises a gene cassette 1, which contains a recombinase recognition sequence, a partial sequence of a drug resistance gene 1 for drug selection reconstituted during translocation recombination, and a drug resistance gene 2 for drug selection after targeting.

The constructs contained in the gene cassette 1 may be, for example, loxP as a recombinase recognition sequence, a neomycin resistance gene as a drug resistance gene 1, and a hygromycin resistance gene as a drug resistance gene 2.

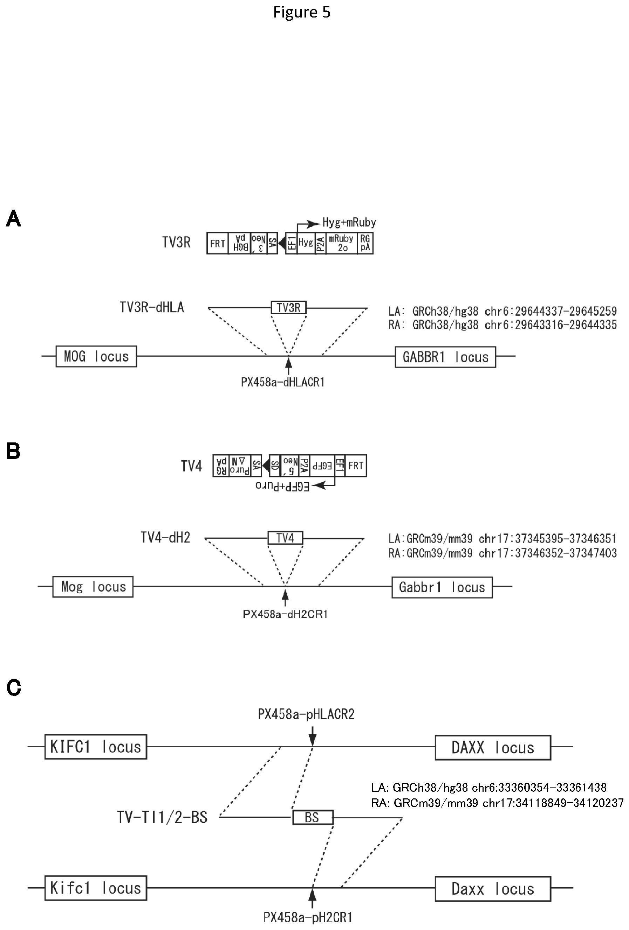

Here, the term "correspond" is used to mean the positional relationship of the sites where recombination will occur. That is, the above-described P1, p1, P2, and p2 are markers indicating the positional relationship of the distal side or proximal side seen from the MHC region as a target to be recombined. Therefore, it is not required that the genes adjacent to P1 and p1, or to P2 and p2, be identical to each other. Depending on the species of mammal, the MHC region may be inverted compared to the orientation of the human MHC region in some cases. In such cases, with regard to the loci on both sides of the site where the targeting vector is incorporated (P1), when the human locus is between the MOG locus and the GABBR1 locus (see Examples and Figure 5), the p1 in an animal with an MHC region inverted can be, for example, between the Kifc1 locus and the Daxx locus.

Figure 5 is a schematic view showing the structure of one aspect of the targeting vector of the present invention. For convenience of explanation, explanation will be made using, as an example, the targeting vector used when a mouse MHC region is substituted with a human MHC region.

In Figure 5A, "TV3R" represents the above-described gene cassette 1. The recombinase recognition sequence is indicated by the horizontal "▲" symbol in the figure, and it is a loxP sequence, etc. In the present invention, "3'neo" is used as a partial sequence of the drug resistance gene 1 for drug selection that is to be reconstituted during translocation recombination (a predetermined partial sequence of the drug resistance gene 1 used in the targeting vector 1). In this case, the drug resistance gene is a neomycin resistance gene. The drug resistance gene 2 for drug selection after targeting is indicated as "Hyg." In this case, the drug resistance gene is a hygromycin resistance gene.

The gene cassette 1 prepared in the Examples of the present invention contains the sequence shown in the following formula (1):

BGHpA-3'Neo-SA-loxP-EF1-Hyg-P2A-mRuby2-RGpA (1)

In the above formula (1), BGHpA represents a poly(A) addition signal sequence derived from bovine growth hormone, 3'Neo represents a partial sequence of the 3' side of the neomycin resistance gene, SA represents a splicing acceptor sequence, loxP represents a recombinase recognition sequence, EF1 represents a promoter sequence derived from human elongation factor 1α, Hyg represents a hygromycin resistance gene, P2A represents a porcine teschovirus-derived 2A sequence, mRuby2 represents a red fluorescent gene, and RGpA represents a poly(A) addition signal sequence derived from rabbit β-globin. However, in the present invention, a Flp recombinase recognition sequence (FRT) can also be added to the cassette 1 shown in (1) above.

"TV3R-dHLA" is the targeting vector 1, and to both sides of TV3R, sequences homologous to the sequences of the regions on both sides of the position (P1), into which TV3R is to be incorporated, are ligated.

In Figure 5A, "PX458a-dHLACR1" is a genome editing vector, and it is a vector that artificially cuts the arbitrary position P1 of the chromosome in order to facilitate recombination via the targeting vector 1. Any known genome editing tool can be used as a genome editing tool utilized in the genome editing vector. Examples of such a genome editing tool may include a tool that utilizes zinc finger nuclease (ZFN), a tool that utilizes tail nuclease (TALEN), and a tool that utilizes CRISPR-Cas9.

PX458a-dHLACR1 is a vector that utilizes CRISPR-Cas9, and is composed of, for example, a guide RNA sequence, a Cas9 sequence, and the gene sequence of the fluorescent protein ametrine.

Figure 1(d) shows an enlarged view of the region from around the MHC region to the distal end. In the Examples of the present invention, in human chromosome, P1 is designated between the MOG locus and the GABBR1 locus, and the targeting vector 1 was designed to be incorporated herein (Figure 5A).

### (ii) Targeting vector 2

In the non-human mammalian chromosome containing an MHC region, targeting vector 2 is incorporated into p1, corresponding to the P1 on the distal side of the non-human MHC region as a target to be recombined.

In the mice of the Examples, the targeting vector 2 induces translocation recombination, between the region from the above-described p1 to the distal end, and the region from P1 to the distal end in human chromosome. The targeting vector 2 comprises a gene cassette 2, wherein the gene cassette 2 contains a recombinase recognition sequence, another partial sequence of the drug resistance gene 1 for drug selection that is to be reconstituted during translocation recombination (another partial sequence other than the predetermined partial sequence of the drug resistance gene 1 used in the target vector 1), and a drug resistance gene 3 for drug selection after targeting.

The constructs contained in the gene cassette 2 may be, for example, loxP as a recombinase recognition sequence, a neomycin resistance gene as a drug resistance gene 1, and a puromycin resistance gene as a drug resistance gene 3.

As described above, depending on the animal species, there are animals with inverted MHC regions. Even in these cases, the targeting vector 2 can be incorporated into the position p1, which corresponds to arbitrary position P1.

In Figure 5B, "TV4" represents the above-described gene cassette 2. The recombinase recognition sequence is indicated by the horizontal "▲" symbol in the figure, and it is a loxP sequence, etc. In the present invention, "5'neo" is used as a partial sequence of the drug resistance gene 1 for drug selection that is to be reconstituted during translocation recombination. In this case, the drug resistance gene is a neomycin resistance gene. The drug resistance gene 2 for drug selection after targeting is indicated as "Puro." In this case, the drug resistance gene is a puromycin resistance gene.

The gene cassette 2 prepared in the Examples of the present invention contains the sequence shown in the following formula (2):

EF1-EGFP-P2A-5'Neo-SD-loxP-SA-T2A-Puro-RGpA (2)

In the above formula (2), EF1 represents a promoter sequence derived from human elongation factor 1α, EGFP represents a green fluorescent gene, P2A represents a porcine teschovirus-derived 2A sequence, 5'Neo represents a partial sequence of the 5' side of the neomycin resistance gene, SD represents a splicing donor sequence, loxP represents a recombinase recognition sequence, SA represents a splicing acceptor sequence, T2A represents a *Thosea asigna* virus-derived 2A sequence, Puro represents a puromycin resistance gene, and RGpA represents a poly(A) addition signal sequence derived from rabbit β-globin. In the present invention, FRT can also be added to the cassette 2 shown in (2) above.

TV4-dH2 is the targeting vector 2, and to both sides of TV4, sequences homologous to the sequences of the regions on both sides of the position (p1), into which TV4 is to be incorporated, are ligated.

In Figure 5B, "PX458a-dH2CR1" is a genome editing vector, and it is a vector that artificially cuts the arbitrary position p1 of the chromosome in order to facilitate recombination via the targeting vector 2. The genome editing tool utilized in the genome editing vector is the same as that described above. PX458a-dH2CR1 is a vector that utilizes CRISPR-Cas9, and is composed of, for example, a guide RNA sequence, a Cas9 sequence, and the gene sequence of the fluorescent protein ametrine.

As with Figure 1(d), in the Examples of the present invention, the non-human chromosome was designed such that the targeting vector 2 was incorporated between the Mog locus and the Gabbr1 locus (Figure 5B).

### < Recombination utilizing genome editing vector >

### (iii) Targeting vector 3

In another aspect of the present invention, in order to carry out the above-described first recombination, a genome editing tool can be utilized, instead of recombination utilizing recombinase. Targeting vector 3 induces translocation recombination between the region from arbitrary position P1, which is on the distal side of the human MHC region as a target to be recombined and is targeted for cleavage by the genome editing tool, to the distal end, in human chromosome 6, and the region from position p1, which is on the distal side of the non-human MHC region and corresponds to the above-described P1, to the distal end, in the chromosome of a non-human mammal in which MHC is present.

Even in this case, the substitution of the MHC region can be performed utilizing translocation recombination from (a) to (b1) and from (b1) to (c) in Figure 1 (two-step recombination 1), or translocation recombination from (a) to (b2) and from (b2) to (c) (two-step recombination 2).

The targeting vector 3 contains a sequence homologous to the sequence of a partial region between the above-described P1 and the above-described human MHC region (this region is referred to as "R1a"), a drug resistance gene 4 for drug selection after targeting, and a sequence homologous to the sequence of a partial region on the distal side of the above-described p1 (this region is referred to as "R2b"), or alternatively, the targeting vector 3 contains a sequence homologous to the sequence of a partial region on the distal side of the above-described P1 (this region is referred to as "R1b"), a drug resistance gene 4 for drug selection after targeting, and a sequence homologous to the sequence of a partial region between the above-described p1 and the above-described non-human MHC region (this region is referred to as "R2a").

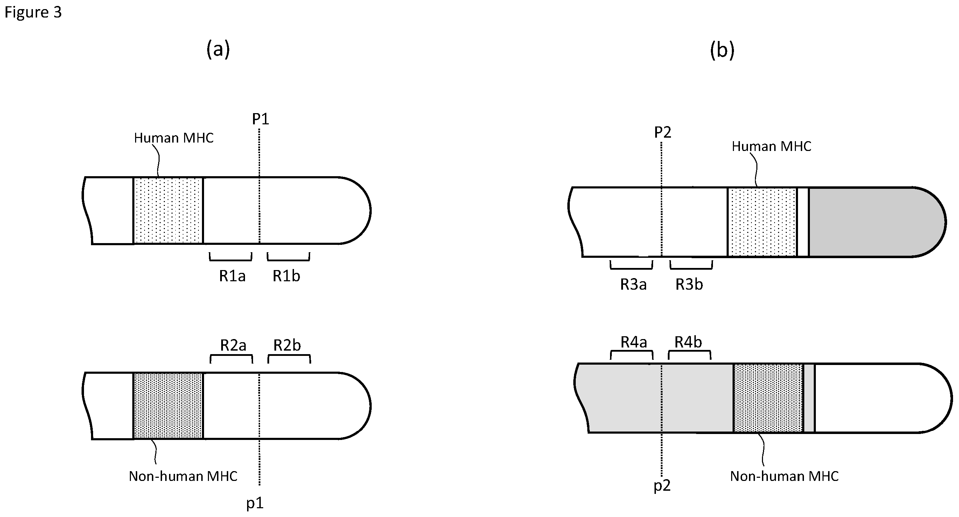

The positional relationship of R1a, R1b, R2a, and R2b is shown in Figure 3(a). It is to be noted that the regions of R1a, R1b, R2a, and R2b may be arbitrary regions seen from the P1 and p1, but are preferably regions located near the P1 and the p1.

The targeting vector 3 used in the present invention can be composed of, for example, an R2a sequence, a CAG promoter sequence, a blasticidin resistance gene, a rabbit β-globin-derived poly(A) addition signal sequence, and an R1b sequence.

(2-2) Second recombination in two-step recombination 1, or first recombination in two-step recombination 2

In the present invention, the vector that causes the second recombination in the two-step recombination 1 and the first recombination in the two-step recombination 2, i.e., the recombination from (b1) to (c) in Figure 1 or the recombination from (a) to (b2) in Figure 1, is referred to as a targeting vector 4.

The recombination in the present section can be performed utilizing a targeting vector utilizing a recombinase, as described above. However, from preliminary experiments conducted by the present inventor, it has been found that the recombination is more efficiently induced when using a targeting vector utilizing a genome editing tool rather than when using a targeting vector utilizing a recombinase, and that subsequent production of non-human mammals is also more effective.

Thus, in the present invention, the second recombination in the two-step recombination 1 or the first recombination in the two-step recombination 2 is performed using a genome editing tool.

### (iv) Targeting Vector 4

In the case of the two-step recombination 1, targeting vector 4 induces translocation recombination between the region from arbitrary position P2, which is on the proximal side of the human MHC region as a target to be recombined and is targeted for cleavage by the genome editing tool, to the distal end, in the chromosome (Figure 1(b1)) after translocation recombination due to the action of the targeting vector 1 and the targeting vector 2, or the action of targeting vector 3, and the region from the position p2, which is on the proximal side of the non-human MHC region and corresponds to the above-described P2, to the distal end, in the chromosome of a non-human mammal in which the non-human MHC is present.

In addition, in the case of the two-step recombination 2, the targeting vector 4 can be used first, and then, the targeting vectors 1 to 3 can also be used. In short, in the recombination shown in Figures 1(a) to 1(c), after the recombination of the region from P1 and p1 to the distal end may be first performed, and the recombination of the region from P2 and p2 to the distal end may be then performed (in the order of Figure 1(a), (b1), and (c)). Alternatively, the recombination of the region from P2 and p2 to the distal end may be first performed, and the recombination of the region from P1 and p1 to the distal end can be then performed (in the order of Figure 1(a), (b2), and (c)).

The targeting vector 4 contains a sequence homologous to the sequence of a partial region R3a on the proximal side of the above-described P2 (between P2 and the centromere), a drug resistance gene 5 (e.g., a blasticidin resistance gene) for drug selection after targeting, and a sequence homologous to the sequence of a partial region R4b between the above-described p2 and the above-described non-human MHC region, or alternatively, the targeting vector 4 contains a sequence homologous to a partial region R3b between the above-described P2 and the human MHC region, a drug resistance gene 5 for drug selection after targeting, and a sequence homologous to the sequence of a partial region R4a on the proximal side of the above-described p2 (between p2 and the centromere).

The positional relationship of R3a, R3b, R4a, and R4b is shown in Figure 3(b). It is to be noted that the regions of R3a, R3b, R4a, and R4b may be arbitrary regions seen from the P2 and p2, but are preferably regions located near the P2 and the p2.

The targeting vector 4 used in the present invention can be composed of, for example, an R3a sequence, a CAG promoter sequence, a blasticidin resistance gene, a rabbit β-globin-derived poly(A) addition signal sequence, and an R4b sequence.

Figure 1(e) shows an enlarged view of the region from around the MHC region to the distal end. In the Examples of the present invention, the targeting vector 4 was designed to induce recombination between the KIFC1 and DAXX loci in human chromosomes and between the Kifc1 and Daxx loci in non-human chromosomes (Figure 5C). The targeting vector 4 contains a blasticidin (BS) resistance gene.

In Figure 5C, "PX458.1a-pHLACR2" is a genome editing vector, and it is a vector that artificially cuts the arbitrary position P2 of the chromosome in order to facilitate translocation recombination performed via the targeting vector 4. PX458.1a-pHLACR2 is a vector that utilizes CRISPR-Cas9, and is composed of, for example, a guide RNA sequence, a Cas9 sequence, and the gene sequence of the fluorescent protein ametrine.

As with PX458.1a-pHLACR2 above, "PX458.1a-pH2CR1" is a genome editing vector, and it is a vector that artificially cuts the arbitrary position p2 of the chromosome in order to facilitate translocation recombination performed via the targeting vector 4. PX458.1a-pH2CR1 is a vector that utilizes CRISPR-Cas9, and is composed of, for example, a guide RNA sequence, a Cas9 sequence, and the gene sequence of the fluorescent protein ametrine.

### < Drug resistance genes for drug selection >

In the present invention, as described above, the drug resistance genes 1 to 5 for drug selection are used for the targeting vectors. The combination of the drug resistance genes 1 to 5 is arbitrary and can be selected, as appropriate, to enable desired drug selection.

Genes used as drug resistance genes 1 to 5 may include a neomycin resistance gene, a hygromycin resistance gene, a puromycin resistance gene, and a blasticidin resistance gene, but are not limited thereto.

< Fluorescent genes for confirming recombination or selection >

In the present invention, a specific fluorescent gene can be included in the targeting vector to confirm chromosome introduction, or to confirm that the introduced chromosome is maintained, or to confirm whether drug selection has been performed as intended. The type of the fluorescent gene can be arbitrarily selected, as long as it allows for confirmation in each step, and different types of fluorescent genes can be used for individual vectors.

Examples of the fluorescent gene may include, for example, green fluorescent genes (GFP, EGFP, etc.), red fluorescent genes, and yellow fluorescent genes.

### (3) Cells

To establish a non-human mammal with a humanized MHC, it is necessary to substitute endogenous MHC genes in non-human mammal cells with human MHC genes.

In the present invention, as described above, a general homologous recombination method or a genome editing technology can be utilized.

Cells are not particularly limited as long as they are animal cells. Examples of the cells may include ES cells, spermatogonial stem cells, and fibroblasts. Among these, ES cells are preferable.

Examples of the medium for cell culture may include, for example, a GMEM medium (Glasgow's Minimal Essential Medium), DMEM (Dulbecco's Modified Eagle's Medium), and RPMI 1640. To the culture medium, commonly used animal cell culture additives, such as fetal bovine serum (FBS), non-essential amino acids, antibiotics (e.g., penicillin, streptomycin, etc.), and growth factors/cytokines (e.g., epidermal growth factor, fibroblast growth factor, leukemia inhibitory factor, etc.), can be added, as appropriate, depending on the cell type.

After culturing the cells for a predetermined period, the cells are harvested by incubating them in a medium containing trypsin. The harvested cells can be passaged multiple times in the presence or absence of feeder cells, as needed. Whether the cultured cells are cells of interest may be confirmed using their marker genes as indicators. When the cells are ES cells, for example, Oct3/4, alkaline phosphatase, Nanog or the like may be used as an indicator, and the ES cells can be detected by any given method such as RT-PCR or Western blotting. Otherwise, the ES cells can also be identified by colony morphology.

### (4) Production of non-human mammals

Non-human mammals can be produced using standard methods such as the production of chimeric animals using ES cells, somatic cell nuclear transfer, intracytoplasmic sperm injection using spermatogonial stem cells, etc.

The non-human mammals as a targets of productio are not particularly limited, and examples thereof may include rodents, livestock animals, and primates. Examples of the rodents may include mice, rats, guinea pigs, and hamsters. Examples of the livestock animals may include bovines, horses, pigs, and sheep. Examples of the primates may include Japanese macaques and common marmosets. Other examples of the non-human animals that can be used herein may include pet animals or laboratory animals, such as dogs, cats, monkeys, and rabbits.

In the present invention, rodents, especially mice, are preferable.

In the case of chimeric animals, the above established ES cells are first aggregated with an early embryo, or were injected into a blastocyst. The thus prepared embryo is called a chimeric embryo. This chimeric embryo is then transplanted into the uterus of a pseudopregnant foster mother, which was then allowed to give birth to produce chimeric animals.

Here, the term "embryo" refers to an individual at any stage in ontogeny from fertilization to birth, and includes early embryos such as a 2-cell stage embryo, a 4-cell stage embryo and an 8-cell stage embryo, a morula stage embryo, and a blastocyst.

Hereafter, for convenience of explanation, ES cells are used as an example in the following explanation.

Methods for producing aggregates using ES cells and embryos may include known methods such as a microinjection method and an aggregation method.

When the microinjection method is adopted, ES cells are injected into recovered embryos to produce cell aggregates. When using the aggregation method is adopted, ES cells may be aggregated with normal embryos from which the zona pellucida has been removed. The ES cells used herein are not particularly limited, and examples thereof may include C57BL/6-derived RENKA, the Y chromosome-shed strain XO of TT2 cells derived from an F1 hybrid between C57BL/6 and CBA, and ES cells established from an F1 hybrid between C57BL/6 and DBA/sCrSlc.

Meanwhile, pseudopregnant female animals to serve as foster mothers can be obtained by breeding normally estrous female animals with male animals castrated by vasoligation or the like. The chimeric embryos prepared by the aforementioned method are transplanted into the uterus of the produced pseudopregnant animals, which are then allowed to give birth, so as to produce chimeric animals.

From these chimeric animals, animals derived from ES cell-transplanted embryos are selected. The selected chimeric animals are then bred with animals from an inbred strain. When the resulting offsprings have exhibited the coat color of the animal derived from the ES cells, it can be confirmed that the ES cells have been introduced into the germline of the chimeric animals.

Whether or not the born offsprings have humanized MHC genes can be determined by digesting the DNA with restriction enzymes and then detecting DNA fragments with a size of interest, or by analyzing according to a PCR method.

### 3. Generation of non-human mammalian chromosomes with deleted or disrupted MHC genes, and cells containing these chromosomes in homozygous or heterozygous forms

In MHC-humanized (heterozygous) ES cells, the MHC region of one allele is humanized. Using a mouse as an example of a non-human mammal 1, CRISPR/Cas9-mediated genomic sequence deletion induction or gene disruption, which targets sequences within the mouse MHC region, will all occur on alleles having the mouse MHC region (Figure 17). Therefore, mouse MHC genes-deficient alleles can be produced by disrupting or deleting the mouse MHC genes in MHC-humanized (heterozygous) ES cells. Since MHC-humanized (heterozygous) mice can be produced from MHC-humanized (heterozygous) ES cells, it is possible to produce mouse MHC genes-deficient mice, if mouse MHC genes-deficient MHC-humanized (heterozygous) ES cells are produced.

In the Examples, using MHC-humanized (heterozygous) ES cells, in which the MHC region ranging from Daxx to Mog was humanized in XO ES cells derived from CBAxC57BL/6 F1 mice, mouse MHC genes-deficient alleles were generated. First, considering distribution of MHC genes on the genome, it was found that 34 of the 39 genes were present in five clusters without non-MHC genes (Figure 18). These clusters are designated d1, d2, d3, d4, and d5, in descending order of size.

Five genes, H2-K1, H2-Oa, H2-M5, H2-M3, and H2-M2, are found to exist independently, without forming a cluster. These isolated MHC genes are classified into d6.

In the present invention, in order to delete or disrupt MHC genes, regarding the MHC genes d1 to d5, the sequences are deleted using two CRISPR/Cas9 designed to sandwich the entire or a part of all the MHC gene loci within the cluster, and for the isolated MHC genes classified into d6, CRISPR/Cas9 designed to disrupt each MHC gene can be used. For example, in order to delete the d1 MHC gene cluster, CRISPR/Cas9 is designed to target both sides of the d1 cluster, i.e., the region between the H2-M10.2 locus and d3 located closest to the centromere side within the d1 cluster in Figure 18, and the region between the H2-M10.6 locus and d6 (H2-M5 gene located closest to the telomere side within the d1 cluster, thereby inducing deletion. CRISPR/Cas9 is designed similarly for other clusters.

Since the nucleotide sequences of genomes upstream and downstream of each MHC cluster often are different between the non-human mammals 1 (e.g., mice) and the mammals 2 (e.g., humans), in the designing of CRISPR/Cas9, these different nucleotide sequences are targeted. Thereby, only the mouse MHC genes (MHC genes 1) can be disrupted or deleted from MHC-humanized heterologous cells.

As described above, regarding the clusters d1 to d5, CRISPR/Cas9 is designed to sandwich each cluster. Moreover, when individual gene clusters d1 to d6 are deleted or disrupted, the number of gene clusters deleted or disrupted at one time is not limited to one, but two or more clusters can also be combined. For example, the gene clusters can be combined with each other, as appropriate, such as a combination of d1 and d3 or a combination of d2 and d3 (two-cluster combination), a combination of d1, d3 and d2 (three-cluster combination), and a combination of d5 and MHC genes classified into d6 (a combination of one cluster and isolated MHC genes).

When the gene clusters d1 to d6 are deleted or disrupted for each gene cluster, what gene cluster is first disrupted or deleted, namely, the order of disruption or deletion is arbitrary. For example, it is possible that d1 is deleted, d2 is then deleted, and d3 is then deleted, so that d1 to d6 may be deleted or disrupted successively, or alternatively, it may also be possible that the gene clusters are deleted regardless of the order. The clusters to be deleted or disrupted may be partial or all.

Otherwise, a single cluster can be divided into subgroups and is then subjected to multiple times of deletion or disruption steps. For example, in the cluster d1 shown in Figure 18, H2-M10.2 to H2-M11 can be divided into a subgroup called dla, and H2-M9 to H2-M10.6 can be divided into a subgroup called d1b, and the clusters in each subgroup can be deleted or disrupted.

On the other hand, since the genes classified as d6 does not form a cluster, CRISPR/Cas9 is designed to disrupt each gene. The d6 MHC genes can be divided into subgroups and can be disrupted separately.

Herein, there is a case where some MHC genes from the non-human mammal 1 (e.g., a mouse) remain in chromosome containing the MHC region of the mammal 2 (e.g., a human). In such a case, the entire MHC genes is disrupted or deleted from the wild-type allele, and at the same time, the remaining non-human mammal 1 MHC genes in the chromosome containing the mammal 2 MHC region are disrupted. In Figure 18, in addition to the human MHC region, the mouse-derived H2-M3 and H2-M2 genes remain, so these remaining genes are also disrupted or deleted.

In this way, all mouse MHC genes can be deleted by deleting or disrupting the MHC gene groups d1 to d6. In the present invention, the clusters d1 to d6 can be arbitrarily selected, and some of these clusters can be deleted or disrupted, or all of the clusters d1 to d6 can also be deleted or disrupted.

In one aspect of the present invention, as shown in Figure 19, MHC genes contained in individual clusters d1 to d6 are sequentially deleted, so that d1 to d6 clones are isolated. The d1 clone is a clone in which the MHC genes in the d1 cluster of MHC-humanized (heterozygous) ES cells have been deleted. The d2 clone is a clone in which the MHC genes in the d2 cluster have been deleted from the d1 clone. The d3 clone is a clone in which the MHC genes in the d3 cluster have been deleted from the d2 clone. The d4 clone is a clone in which the MHC genes in the d4 cluster have been deleted from the d3 clone. The d5 clone is a clone in which the MHC genes in the d5 cluster have been deleted from the d4 clone, and further, the H2-M5 gene has been simultaneously disrupted. The d6 clone is a clone in which the H2-K1, H2-Oa, H2-M3, and H2-M2 genes of the d5 clone are disrupted. Since, in generation of the d6 clone, simultaneous disruption of the four genes reduces gene disruption efficiency, in some cases, the d6a clone was isolated by disrupting only the H2-K1 and H2-M3 genes of the d5 clone, and then, the H2-Oa and H2-M2 genes were disrupted to isolate the d6 clone.

### 4. Non-human mammals having chromosomes in which MHC genes are deleted or disrupted

As described above, non-human mammals can be produced using ES cells with chromosomes in which the MHC genes 1 is entirely deleted or disrupted. For example, non-human mammals can be produced by transplanting ES cells with chromosomes, in which the MHC genes 1 is entirely deleted or disrupted, into early embryos, or by performing somatic cell nuclear transfer of ES cells with chromosomes, in which the MHC genes 1 is entirely deleted or disrupted.

Furthermore, such chromosomes, in which the MHC genes 1 is deleted or disrupted, can be introduced from the above-described cells into other cells by a microcell-mediated chromosome transfer method, etc.

### Examples

Hereinafter, the present invention will be more specifically described in the following examples. However, these examples are not intended to limit the scope of the present invention.

### [Example 1]

### 1. Generation of targeting vectors

### < Methods >

### Methods

### Generation of TV3R-dHLA

pEF-GFP (Addgene plasmid #11154; http://n2t.net/addgene:11154; RRID: Addgene_11154) was digested with restriction enzymes, EcoRI-HF (New England BioLabs) and NotI-HF (New England BioLabs), and a hygromycin resistance gene was cloned downstream of the human elongation factor 1 α-derived promoter sequence, using the In-Fusion^{®} HD Cloning Kit (Clontech) according to the protocols included therewith, so as to generate pEF-Hyg (SEQ ID No: 1). Subsequently, the upstream side of the human elongation factor 1α-derived promoter sequence encoded by pEF-Hyg was digested with the restriction enzyme SalI, and the synthetic DNA fragment FRT-BGHpA-3'Neo-SA-loxP (Thermo Fisher Scientific) (SEQ ID No: 2) was cloned using the In-Fusion^{®} HD Cloning Kit, so as to generate TV3.

Next, a sequence containing the 5'-homologous region and 3'-homologous region of the targeting vector TV3R-dHLA was amplified using the MRC5 cell genome as a template, also using the following primer set, and employing PrimeSTAR^{®} GXL DNA Polymerase (Takara) according to the protocols included therewith.
hMOG CR LA fw1: AATTAGCCATGTGTGGTGGCACACG (SEQ ID No: 3)
hMOG CR RA rv1: CCCTGAGAGTCCTGTGCATATCAGC (SEQ ID No: 4)

Furthermore, the entire homologous region was amplified by a nested PCR method using the following primer set, and was cloned into pPGEM^{®}-T Easy Vector (Promega) to generate pGEMTe-dHLA.
hMOG nest fw1: TGAGGCAGGAGAATCACTTG (SEQ ID No: 5)
hMOG nest rv1: GCTAAGGATTAAATCACTGCGGTTATC (SEQ ID No: 6)

Next, pGEMTe-dHLA was amplified using the following primer set and employing PrimeSTAR^{®} GXL DNA Polymerase. TV3 was digested with restriction enzymes, SalI-HF (New England BioLabs) and HindIII-HF (New England BioLabs), to prepare a fragment containing FRT-BGHpA-3'Neo-SA-loxP. This fragment was cloned using the In-Fusion^{®} HD Cloning Kit to generate TV3-dHLA.
TV3-dHLA inf fw1:
   GACCTGCAGCCCAAGCTACCAGGGATAACAGGGGAACAG (SEQ ID No: 7)
TV3-dHLA inf rv1:
   GAATAGGAACTTCGTCGACTGTCAAGCAGCTAGCAGGC (SEQ ID No: 8)

Furthermore, the synthetic DNA fragment P2A-mRuby2 (SEQ ID No: 9) (Thermo Fisher Scientific) was amplified using the following primer set and employing PrimeSTAR^{®} GXL DNA Polymerase. The amplified product and TV3-dHLA were digested with the restriction enzyme BssHII (New England BioLabs), and both products were then ligated using a Ligation-Convenience Kit (Nippon Gene) to generate TV3R-dHLA.
RGpA seq fw1: ACTACTCCCAGTCATAGCTG (SEQ ID No: 10)
BGHpA seq rv1: CTATTGTCTTCCCAATCCTCC (SEQ ID No: 11)

### Generation of TV4-dH2

pEF-GFP (Addgene plasmid #11154; http://n2t.net/addgene:11154; RRID: Addgene_11154) was digested with the restriction enzymes EcoRI-HF and NotI-HF, and an EGFP gene was cloned downstream of the human elongation factor 1α-derived promoter sequence, using the In-Fusion^{®} HD Cloning Kit (Clontech) according to the protocols included therewith, so as to generate pEF-EGFP2 (SEQ ID No: 12). Subsequently, pEF-EGFP2 was digested with the restriction enzyme BsrGI (New England BioLabs), and then, into this digested pEF-EGFP2, the sequence P2A-5'Neo-SD-loxP-SA-T2A-Puro (Thermo Fisher Scientific) (SEQ ID No: 13) prepared as two synthetic DNA fragments was cloned using the In-Fusion^{®} HD Cloning Kit, so as to generate TV4dF.

TV4dF was digested with the restriction enzyme SalI-HF, and the following annealed oligo DNA was ligated to this digest using the Ligation-Convenience Kit, so as to generate TV4.
FRT fw: 
TCGAGGAAGTTCCTATTCCGAAGTTCCTATTCTCTAGAAAGTATAGGAACTT
CG (SEQ ID No: 14)
FRT rv:

TCGACGAAGTTCCTATACTTTCTAGAGAATAGGAACTTCGGAATAGGAACT
TCC (SEQ ID No: 15)

Next, a sequence containing the 5'-homologous region and 3'-homologous region of the targeting vector TV4-dH2 was amplified using the C57BL/6 mouse genome as a template, also using the following primer set, and employing PrimeSTAR^{®} GXL DNA Polymerase according to the protocols included therewith.
Mog CR LA fw1: GCGTCAGATCTCATTATGGATGGCTG (SEQ ID No: 16)
Mog CR RA rv1: CGAGCCTATGAGGGTCATTCTCACTC (SEQ ID No: 17)

Furthermore, the entire homologous region was amplified using the following primer set according to a Nested PCR method, and was cloned into pGEM^{®}-T Easy Vector (Promega) to generate pGEMTe-dH2.
Mog nest fw1: GAACTCAGGACCTTCAGAAG (SEQ ID No: 18)
Mog nest rv1: CAACTAACACAGTTACTCCCATAAC (SEQ ID No: 19)

Next, pGEMTe-dH2 was amplified using the following primer set and employing PrimeSTAR^{®} GXL DNA Polymerase. TV4 was digested with the restriction enzymes SalI-HF and HindIII-HF to prepare a fragment containing P2A-5'Neo-SD-loxP-SA-T2A-Puro. This fragment was then cloned using the In-Fusion^{®} HD Cloning Kit to generate TV4-dH2.
TV4-dH2 inf fw1:
   GTATAGGAACTTCGTCGAACGCTGGTAACATTTGCCAACATCT (SEQ ID No: 20)
TV4-dH2 inf rv1:
   ACCTGCAGCCCAAGCTTTGTTGGCCTGATATCTCATTAAATCTG (SEQ ID No: 21)

### Generation of TV-TI1/2-BS

The Hprt expression vector TV-Tl1/2-Hprt (SEQ ID No. 22), which has the pGEM^{®}-T Easy Vector as a vector skeleton and contains the 5'-homologous region and 3'-homologous region to be used in TV-Tl1/2-BS, was digested with restriction enzymes XhoI (New England BioLabs) and XbaI (New England BioLabs). Into the resulting vector, a synthetic DNA fragment (IDT) (SEQ ID No. 23) containing a blasticidin resistance gene was cloned using the In-Fusion^{®} HD Cloning Kit to generate TV-Tl1/2-BS.

### Generation of genome editing vector (1)

The gRNAs of PX458a-dHLACR1 and PX458a-dH2CR1 were designed based on the following sequences. pSpCas9(BB)-2A-GFP (PX458) (Addgene plasmid #48138; http://n2t.net/addgene:48138; RRID: Addgene 48138) was modified, and the EGFP gene was substituted with the fluorescent gene ametrine to generate PX458a (SEQ ID No: 24), which was used for the expression of gRNA and Cas9.
PX458a-dHLACR1: CTAGCTGCTTGACAGTAACC (SEQ ID No: 25)
PX458a-dH2CR1: GATATCAGGCCAACAAACGC (SEQ ID No: 26)

### Generation of genome editing vector (2)

The gRNAs of PX458.1a-pHLACR2 and PX458.1a-pH2CR1 were designed based on the following sequences, and for the expression of gRNA and Cas9, PX458.1a constructed by modifying the gRNA scaffold sequence of PX458a was used (SEQ ID No: 27).
PX458.1a-pHLACR2: GACTCATAAACCGGAGGAGC (SEQ ID No: 28)
PX458.1a-pH2CR1: CGTGTAGTAGGCGCCCGTTA (SEQ ID No: 29)

### Preparation of drug-resistant mouse embryonic fibroblasts (MEFs)

Drug-resistant MEFs were prepared to culture ES cells on feeder cells even during drug selection. First, MEFs were prepared from embryos on embryonic day 14.5, and were then infected with a lentivirus expressing a gene (HBPN) constructed by connecting a hygromycin resistance gene, a blasticidin resistance gene, a puromycin resistance gene and a neomycin resistance gene by a 2A sequence. To improve the proliferative ability of the MEFs, HBPN-expressing MEFs were cultured under hypoxic conditions of 5% oxygen and 5% CO2, and were then treated with mitomycin C, and the resulting MEFs were used as drug-resistant MEFs.

### Cloning of MRC5-A9 fused cells hChA9

MRC5 cells transfected with the Neo resistance gene using a lentivirus were fused with A9 cells using GenomONE-CF (ISHIHARA SANGYO KAISHA, LTD.), and Ouabain-resistant/G418-resistant cells were isolated as fused cells. Human cells were Ouabain-sensitive, while mouse cells were Ouabain-insensitive. Thus, it is possible to select fused cells by adding G418 and Ouabain to the medium. Fused cells having human chromosome 6 were identified by genomic PCR using the following primer set located in the intergenic region of KIFC1 and DAXX.
pHLA probe fw2: AGTAAATCCTGACTGAGCACCTCCTG (SEQ ID No: 30)
pHLA probe rv1: GCTTGCGTGAGGCTGAAGTGTC (SEQ ID No: 31)

Moreover, for the detection of human chromosome 6 by FISH, a DNA fragment artificially synthesized by gathering sequences specific to the human chromosome 6 centromere was used in preparation of a probe, with reference to the report by Jabs et al. (Am. J. Hum. Genet. 41:374-390, 1987).

The synthesized sequence is as follows.

AAGGAGTTTCTGAGATTGCTTCTGTCTAGCTTTTATGGAAAGATATTTCCTTT
TCTACCATAGGCCTCAAAGCGCTCTTAGTATACACTTCCAAATTCTACAAAG
AGAGTGTTACTAAACCGCTCTCTCAAAGGAAATGTTAAACTCTATGAGTTG
AAACACAGCACACAAACAGTTCTGAGACACTTCTGTCTGCCTTTTATGTGAA
GACATCCCTTTCCAAAGAATGCCTCCAAGGGTTCAAAATATCCACTTGTAG
ACTTTACAAAGAGAGTGTTCAAAACTTTCTCTACCAAAAGAAAGGTTAAAG
ACGGTGAGTTCAAACGCACACATCACAAAGTTGTTTCTAAGAATCATCTAC
TATGTTCTAGAGATGTTCCTTTCTATCATAGCTCAATGTCTAAATATCCACT
GAATC (SEQ ID No: 32)

### Cloning of TV3RhChA9

A clone (TV3RhChA9) that targets TV3R-dHLA to MRC5-A9 fused cells was produced by transfecting hChA9 with PX458a-dHLACR1 and the targeting vector TV3R-dHLA, using PEI max (Polysciences). PX458a-dHLACR1 was co-transfected in order to increase the homologous recombination efficiency of the targeting vector. The day after the transfection, vector-transfected cells were sorted using the expression of the fluorescent gene mRuby2 on TV3R-dHLA as an indicator, and mRuby2-positive, hygromycin-resistant clones were isolated. Genomic DNA was prepared from the isolated clones, and recombinant clones were then identified by PCR. The primers used in PCR are as follows. Even when the recloning of the isolated clones was carried out, it was analyzed in the same manner.
dHLA 5 typing fw1:CTTGGGGTCCAGAGAAGAAAATCACTC (SEQ ID No: 33)
BGHpA typing fw1: CTCTATGGCTTCTGAGGCGGAAAGAAC (SEQ ID No: 34)

### Cloning of TV4RENKA

TV4RENKA is an ES cell clone prepared by transfecting RENKA with PX458a-dH2CR1 and the targeting vector TV4-dH2 using Lipofectamine 3000 (Thermo Fisher Scientific). PX458a-dH2CR1 was co-transfected to increase the homologous recombination efficiency of the targeting vector. EGFP-positive clones were isolated, and recombinant clones were then identified by genomic PCR. For XO and BDF1, clones can also be isolated in the same manner as described above.

The primers used in PCR are as follows.
Mog 3' typing fw1:CGGAGCCTCACGCGATGATCTA (SEQ ID No: 35)
Mog 3' typing fw2: AGTACACTGTAGTTGTCCTCAGATACTCC (SEQ ID No: 36)
Mog 3' typing rv1: TCCACCCCAAACATTTCCACTAACTG (SEQ ID No: 37)

### Cloning of TV3-4RENKA

TV3-4RENKA is an ES cell clone prepared by introducing modified human chromosome 6 from TV3RhChA9 into TV4RENKA using a retro-MMCT method (Suzuki, T. et al. (2016) PloS One, 11(6): e0157187.). Cells that were mRuby2-positive and hygromycin-resistant were cloned, and clones with a chromosome count of 41 was then isolated.. In FISH analysis, detection was carried out using Cy3-labeled mouse Cot-1 and Cy5-labeled RP11-54H13.

### Cloning of TV3-4RENKATl3/4

A Cre expression vector was introduced into TV3-4RENKA using Lipofectamine 3000, and chromosomal translocation was then induced between the loxP sites. Clones, in which chromosomal translocation had occurred, were isolated using G418 resistance as an indicator, and such clones were designated "TV3-4RENKATl3/4." After confirming the occurrence of recombination by genomic PCR, it was confirmed by FISH analysis that translocation had occurred between mouse chromosome 17 and human chromosome 6.

The primers used in PCR are as follows.
5Neo_O fw1: TGCCAGGCCAGGATCTGCTG (SEQ ID No: 38)
Neo_O rv1: ATCCGGTGCTTGGCTTGATG (SEQ ID No: 39)

In the FISH analysis, detection was carried out using Cy3-labeled RP11-54H13, DY490-labeled RP23-147G23 and Cy5-labeled RP23-119G24, or Cy3-labeled human Cot-1 and DY490-labeled mouse Cot-1.

### Cloning of TV3-4RENKATlb

In the cloning of TV3-4RENKAT1b, TV-T11/2-BS, PX458.1a-pHLACR2, and PX458.1a-pH2CR1 were co-transfected into TV3-4RENKAT13/4, using Lipofectamine 3000. The day after the transfection, ametrine-positive cells were sorted, and blasticidin-resistant clones were then isolated. After confirming the occurrence of recombination by genomic PCR, it was confirmed by FISH analysis that substitution of the MHC region occurred.

The primers used in PCR are as follows.
pH2 seq fw: CACATGTCCCTTCTGCCATAAG (SEQ ID No: 40)
Tlb typing rv1: AGAGATCACTGAAGCTGCTAC (SEQ ID No: 41)

In the FISH analysis, detection was carried out using Cy3-labeled RP11-147G23, DY490-labeled human Cot-1, and Cy5-labeled RP23-306H20.

### Cloning of TV3-4RENKATlbdh6

In order to isolate TV3-4RENKATlbdh6, in which the remaining human chromosome 6 had been shed from TV3-4RENKATlb, mRuby2-negative cells were sorted and were then seeded on feeder cells to isolate clones. Furthermore, FISH analysis was carried out on clones having a chromosome count of 40 (39 in the case of XO) based on the karyotype analysis, it was confirmed that the remaining human chromosome 6 had been shed. In the FISH analysis, detection was carried out using Cy3-labeled RP11-147G23, DY490-labeled human Cot-1, and Cy5-labeled RP23-361C19. TV3-4XOTlbdh6 and TV3-4BDF1Tlbdh6 were also isolated using the same procedures as those for TV3-4RENKATlbdh6.

### 2. Production of MHC-humanized mice

MHC-humanized chimeric mice were produced by a chimera aggregation method using MHC-humanized ES cells and ICR-derived embryos. In the case of chimeric mice produced using RENKA-derived MHC-humanized ES cells, MHC-humanized mice were established by isolating spermatids from the testes and then injecting them into eggs from B6/DBA F1 or ICR mice by intracytoplasmic sperm injection. On the other hand, in the case of female chimeric mice produced using XO ES cell-derived MHC-humanized ES cells, MHC-humanized chimeric mice (MHC-humanized heterozygous mice) were established by breeding the female mice with C57BL/6 male mice. The establishment of MHC-humanized mice was confirmed by the EGFP fluorescence of the offsprings and the genomic PCR method.

The primer set used for genomic PCR is as follows.
DAXX:
   Fw, TCTAGTCCCTTCAAGGGCTGAG (SEQ ID No: 42)
   Rv, ACAATGTCTCTCTGAAGGCTGTAC (SEQ ID No: 43)
MICA:
   Fw, AACATCACCGTGACATGCAG (SEQ ID No: 44)
   Rv, TGTCTGCCAATGACTCTGAAG (SEQ ID No: 45)
MOG:
   Fw, CAGCTGCAGCAATTACCGGAGTG (SEQ ID No: 46)
   Rv, GAAGGGAGGCATGTCAGTAGGTC (SEQ ID No: 47)
S76:
   Fw, CCTTTGTGACAGCGCACGTT (SEQ ID No: 48)
   Rv, CCAGACACTAGGGCTTTCGT (SEQ ID No: 49)
DAXX (confirmation using 3 primers):
   Human/mouse common primer, CGGCTGGATGAGGTCATCTCCAA (SEQ ID No: 50)
   Mouse-specific primer, GACTCAACTCTGGGAGCTCATG (SEQ ID No: 51)
   Human-specific primer, GTTTCAAACAGGTGGCTCATGC (SEQ ID No: 52)

### 3. Results and consideration

### Construction of vectors for substitution of MHC regions

First, vectors necessary for a series of recombination operations were constructed. Considering the influence of translocation substitution on the expression of neighboring genes, the targeting vectors TV3R-dHLA and TV4-dH2 used for recombinant introduction of a TV3R sequence or a TV4 sequence between the MOG and GABBR1 loci, which have a relatively large intergenic sequence on the distal side of the MHC region in humans or mice, were designed (Figures 5A and B). Moreover, in order to increase the homologous recombination efficiency, the CRISPR vectors PX458a-dHLACR1 and PX458a-dH2CR1, which induce DNA double-strand cleavage to each target site, were produced.

TV3R encodes a loxP sequence necessary for translocation induction, a 3' sequence of a Neo resistance gene for G418 resistance during translocation recombination, an FRT sequence for removing sequences within the targeting vector after translocation induction, a hygromycin resistance gene for drug selection during targeting, and a red fluorescent gene mRuby2 for detecting the presence of targeted chromosomes.

On the other hand, TV4 encodes a loxP sequence necessary for translocation induction, a 5' sequence of a Neo resistance gene for G418 resistance during translocation recombination, an FRT sequence for removing sequences within the targeting vector after translocation induction, a puromycin resistance gene for drug selection during targeting, and a green fluorescent gene EGFP for detecting the presence of targeted chromosomes. The TV3R sequence and the TV4 sequence were designed, such that the EGFP expression unit and the reconstituted Neo resistance gene would remain in in mouse chromosome 17 after translocation induction, so that MHC-humanized mouse chromosome 17-retaining cells can be selected using drug and fluorescence.

Next, the targeting vector TV-Tl 1/2-BS used to induce translocation recombination between the DAXX and KIFC1 loci, which have a relatively large intergenic sequence on the proximal side of the MHC region in humans and mice, and the CRISPR vectors PX458.1a-pHLACR2 and PX458.1a-pH2CR1 used to excise the respective target sites and increase recombination efficiency, were produced (Figure 5C). Since TV-Tl 1/2-BS contained a blasticidin resistance gene expression cassette, this vector was designed to select the recombined and introduced cells by blasticidin resistance. Moreover, the vector was designed, such that the blasticidin resistance gene could be recombined and introduced onto the remaining human chromosome 6, which would be ultimately removed from ES cells. Thereby, since the foreign gene expression cassette is not introduced into the proximal side of the MHC region of MHC-humanized mouse chromosome 17, it is considered that the influence on gene expression around the humanized MHC region can be minimized.

### Generation of A9-MRC5 fused cells

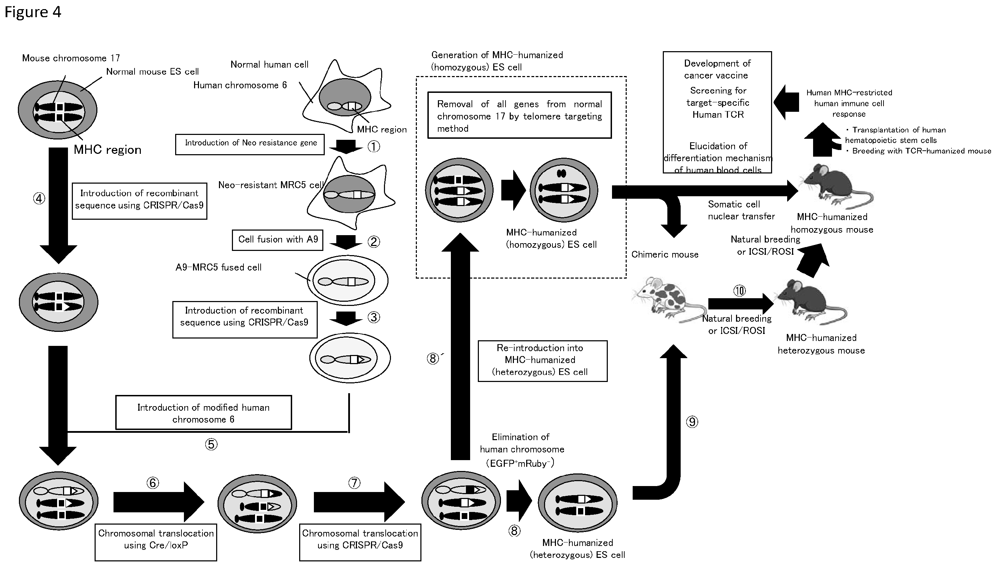

To introduce human chromosome 6 from normal human fibroblasts (MRC5 cells) into mouse ES cells (hereinafter referred to as ES cells), it is necessary to generate fused cells of MRC5 cells with mouse fibroblasts A9 having high chromosome-donating ability. Thus, to select the fused cells by drug selection, a neomycin resistance gene was first introduced into the MRC5 cells using lentivirus (Figure 4, Step 1 (circled number 1; hereafter, from Step 1 onward, each step is numbered using the same circle number as Step 1)). Next, the neomycin-resistant MRC5 cells were fused with the A9 cells, and the fused cell line hChA9, which was resistant to both ouabain and G418, drugs that selectively kill human cells, was isolated (Figure 4, Step 2).

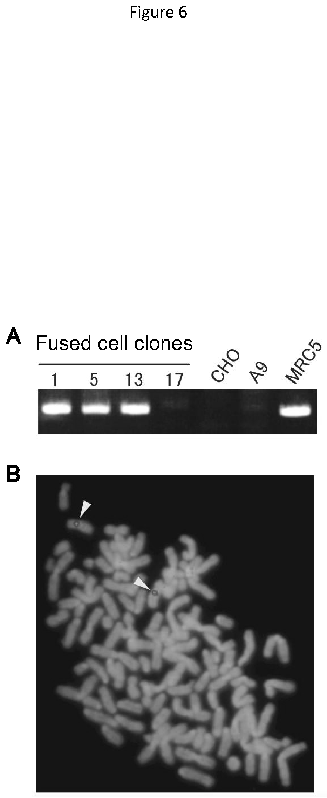

Whether or not sequences within the human HLA region are present in the isolated clones was confirmed by genomic PCR. As a result, signals were detected in clones #1, 5, and 13 (Figure 6A). Furthermore, in order to confirm that human chromosome 6 is present in hChA9 #5, analysis was carried out by a FISH method using a probe specific for the sequence of the human chromosome 6 centromere, and as a result, it could be confirmed that this fused cell clone contained two human chromosome 6 strands (Figure 6B).

### Targeting of TV3R

The targeting vector TV3R-dHLA was recombined and introduced into the distal side of the MHC region of human chromosome 6 of hChA9 #5, so that the sequence necessary for substitution of the MHC region was introduced (Figure 4, Step 3).

As a result, hygromycin-resistant clones were isolated and were analyzed by genomic PCR, so that multiple targeted cells, TV3RhChA9, could be isolated (Figure 7A). To eliminate the possibility of clone contamination, TV3RhChA9 #5-45 (128) was recloned to isolate TV3RhChA9 #5-45a (128) (Figure 7B). FISH analysis was performed on TV3RhChA9 #5-45a (128), and as a result, it could be confirmed that a majority of cells contained a single human chromosome 6 strand (Figure 7C). Thus, TV3RhChA9 #5-45a (128) was used as modified human chromosome. 6-donating cells.

### Targeting of TV4

TV4-dH2 was targeted to the distal side of the MHC region present on chromosome 17 of the B6-derived mouse ES cell RENKA, so that the sequence necessary for swapping of the MHC region was introduced (Figure 4, Step 4).

EGFP-positive clones were analyzed by genomic PCR, and as a result, a large number of recombined and introduced clones could be confirmed (Figure 8A). Since EGFP within the TV4 sequence can be utilized to confirm germline transmission if it is present only in the MHC-humanized allele, the clone TV4RENKA #17, which has a chromosome count of 40 and in which only one allele is targeted, was used in subsequent experiments (Figure 8)

### Introduction of modified human chromosome 6

The modified human chromosome 6 of TV3RhChA9 #5-45a (128) was introduced into TV4RENKA #17 (Figure 4, Step 5). In general, ES cells have low chromosome introduction efficiency, and also, fused cells tend to have lower chromosome donation ability than A9 cells. Thus, it may be considered that chromosome introduction is not possible using the conventional chromosome introduction method using polyethylene glycol (PEG-MMCT method). Therefore, a highly efficient chromosome introduction method (retro-MMCT method) utilizing ecotropic envelope proteins derived from murine leukemia virus was applied to the introduction of the modified human chromosome 6.

ES cells that were hygromycin-resistant and mRuby2-positive were cloned, and chromosome preparations were prepared to carry out karyotype analysis. While normal mouse cells have a chromosome count of 40 (39 in XO), TV3-4RENKA #17/5-45a-3 could be confirmed to have a chromosome count of 41 (40 in XO). Moreover, since these cells could be confirmed to retain submetacentric chromosome, which is characteristic of human chromosome 6, FISH analysis was further carried out. As a result, this chromosome was confirmed to contain the human MHC region. Therefore, it was considered that the introduction of the modified human chromosome 6 was successfully carried out (Figure 9).

### Induction of chromosomal translocation using Cre/loxP

Cre recombinase can induce translocation between loxP sites on independent chromosomes. Thus, in order to induce translocation between the modified human chromosome 6 retained by TV3-4RENKA #17/5-45a-3 and the distal side of the MHC region of modified mouse chromosome 17, Cre recombinase was allowed to transiently express in TV3-4RENKA #17/5-45a-3 (Figure 4, Step 6). When translocation recombination is induced between the loxP sites present in the TV3 sequence and the TV4 sequence, the Neo resistance gene is reconstituted (Figure 10A), and thus, G418-resistant clones were isolated. It could be confirmed by genomic PCR and FISH method that translocation recombination had occurred in clones with a chromosome count of 41 (40 in XO) among these clones. This clone was designated "TV3-4RENKA Tl3/4 #17-1," and was used in subsequent experiments (Figures 10B-D).

### Induction of chromosomal translocation using CRISPR/Cas9

Since translocation of the proximal side of the MHC region was planned to be induced using the recombinase Dre, as in the case of the distal side, ES cells, in which the Dre recognition sequence rox had been recombined and introduced into the proximal side, were initially prepared. However, Dre-mediated translocation had low efficiency, and also, the expression of the HPRT gene, which was intended to be used as a selection marker, was highly demanding in ES cells. Therefore, it was found that the expression of the HPRT gene reconstituted by recombinant translocation could not withstand selective culture with HAT. Accordingly, the modification of mouse chromosome 17 was changed to only introduction of TV4, disruption of the Hprt gene in ES cells was canceled, and TV3-4RENKA Tl3/4 #17-1 was produced.

Hence, translocation was induced to the proximal side of the MHC region using CRISPR/Cas9. Specifically, a CRISPR vector that cuts the KIFC1-DAXX intergenic region of mouse chromosome 17 and human chromosome 17, respectively, and the translocation induction targeting vector TV-Tl1/2-BS, were introduced into TV3-4RENKA Tl3/4 #17-1, clones that were resistant to blasticidin were then isolated (Figure 4, Step 7). (Figure 4, Step 7). It could be confirmed by genomic PCR that, among these clones, the clone TV3-4RENKA Tlb #38 had a chromosome count of 41 (40 in XO), and that translocation recombination occurred. Moreover, it was found by FISH analysis that modified human chromosome 6 was translocated with modified chromosome 17, but not with wild-type chromosome 17. Therefore, TV3-4RENKA Tlb #38 was used in subsequent experiments (Figure 11).

### Isolation of MHC-humanized ES cells

To ensure undifferentiation of the MHC-humanized ES cells, initially, the MHC-humanized mouse chromosome 17 produced thus far had been planned to be introduced into low-passage ES cells, and endogenous mouse chromosome was deleted to generate the MHC-humanized ES cells (Figure 4, Step 8'). However, to perform these operations, production of ES-A9 fused cells and introduction of MHC-humanized chromosome into ES cells were required, and it was found that chromosome abnormalities frequently would occur during this process, and thus, the operations were restricted. Thus, an attempt was made to produce chimeric mice by isolating a large number of MHC-humanized ES clones from TV3-4RENKA Tlb #38 by spontaneously shedding human chromosomes (Figure 4, Step 8).

It has been reported that human chromosomes are easily shed in mouse cells. Therefore, TV3-4RENKA Tlb #38 was cultured in the absence of a selective drug for several days to induce the shedding of the remaining human chromosome 6. To isolate the remaining human chromosome 6-shed clones, it was initially planned to utilize 6-thioguanine capable of selecting HPRT gene-deficient cells, as a selective drug. However, as mentioned above, it was found difficult for the Hprt gene to be used as a selection marker in ES cells.

Thus, clones were isolated by sorting mRuby2-negative cells, using the expression of the fluorescent gene mRuby2, which was designed to remain on the remaining human chromosome 6, as an indicator. As a result, the TV3-4RENKATlb #38dh6 clones, which shed the remaining human chromosome 6, were successfully isolated (Figure 12). Among these, the TV3-4RENKA Tlbdh6 #3, which had a chromosome count of 40 (39 in XO) and in which the MHC-humanized mouse chromosome 17 could be confirmed by the FISH method, was used in subsequent experiments. Moreover, using the mouse genomic sequence and sequences within the human MHC region as references, the whole genome sequencing analysis of the TV3-4RENKA Tlbdh6 #3 was performed, and the haplotype of the introduced human MHC genes was identified (Figure 12C).

### Establishment of MHC-humanized mice

Chimeric mice were produced from TV3-4RENKATlb #38dh6 #3, and three mice with a coat color chimerism percentage of 50% to 60% were obtained (Figure 13A) (Figure 4, Step 9).

ES-derived cells having MHC-humanized chromosomes are designed to express EGFP (Figure 13B). Thus, the contribution percentage of MHC-humanized ES cells in the testes was analyzed using EGFP expression as an indicator, and as a result, it was found that nearly 100% were EGFP-positive (Figure 13C). This suggested that intracytoplasmic sperm injection (ICSI/ROSI) of testicular cells would likely result in offsprings. Therefore, intracytoplasmic sperm injection was performed using testicular cells from chimeric mice (Figure 4, Step 10).

As a result, MHC-humanized mice with a whole body that glows green were successfully obtained due to germline transmission of MHC-humanized ES cells (Figures 14A and B). Furthermore, it was also confirmed by genomic PCR that EGFP-positive individuals possessed human MHC region sequences (Figure 14C). From these results, it was found that MHC-humanized (heterozygous) mice were successfully established.

### Production of MHC-humanized mice by natural breeding of chimeric mice

From the previous results, it was found that MHC-humanized chimeric mice generated from B6-derived ES cells had low fertility. Thus, humanization of the MHCs of XO ES cells (B6/CBA F1) that were to develop as females was also carried out, so as to produce chimeric mice (Figure 15A) (Figure 4, Step 9). The produced chimeric mice were then bred with wild-type mice.

As a result, MHC-humanized heterozygous mice were successfully obtained by natural breeding (Figures 15B-D) (Figure 4, Step 10).

### 4. Analysis of expression of human MHC genes in MHC-humanized (heterozygous) mice

### < Methods >

### Analysis of expression of human MHC (also referred to as HLA) protein

For preparation of splenocytes, an excised spleen was first minced with a knife, and the tissue section was then mashed using the frosted processing part of a glass slide. Subsequently, the cells were hemolyzed with 0.83% ammonium chloride and were then passed through a nylon mesh to prepare splenocytes. For flow cytometry analysis, the cells were stained with a Pacific Blue-labeled anti-mouse CD19 antibody (Biolegend), or with an APC-labeled anti-HLA class I antibody (MBL) or APC-labeled anti-HLA-DR, DP, DQ antibody (Biolegend) that had been labeled using the APC Conjugation Kit-Lightning-Link (Abcam), and were then analyzed using a FACS AriaIII (BD). In the analysis according to a Western blotting method, detection was carried out using anti-HLA-A, B, C antibody (MBL), anti-HLA-A antibody (Abcam), anti-HLA-DRA antibody (Abcam), or HRP-labeled anti-ACTB antibody (Genscript).

For preparation of lung-derived fibroblasts, an excised lung tissues were first minced with a knife and treated with 0.1% collagenase A (Roche) for 1 hour. Cells were released by pipetting and then passed through a nylon mesh to prepare lung-derived fibroblasts. The lung-derived fibroblasts were cultured in DMEM supplemented with 10% FCS.

### Results

The expression of human MHC genes in splenocytes was analyzed at the protein level. First, splenocytes were prepared from MHC-humanized (heterozygous) mice (Het) established from wild-type (Wt) and XO ES cells. The expression of human MHC proteins was analyzed by a Western blotting method using an anti-HLA-A, B, C, antibody, an anti-HLA-A antibody, and an anti-HLA-DRA antibody. As a result, the expression of human MHC was specifically detected in Het-derived splenocytes (Figure 16A).

Next, the expression of HLA proteins on the cell surface was analyzed. Splenocytes are primarily composed of B cells and T cells, and MHC class I should be expressed in all cells, while MHC class II should be expressed in B cells. Thus, flow cytometry analysis was performed using an antibody against the B cell marker CD19, an anti-HLA class I antibody that recognizes human MHC class I, and an anti-HLA-DP, DQ, DR antibody that recognizes human MHC class II. As a result, HLA class I was detected in all Het-derived splenocytes, while it could be confirmed that HLA-DP, DQ, DR were specifically expressed in Het-derived B cells (Figure 16B).

### [Example 2]

### Production of cells and animals having MHC region-deleted and -deficient chromosome

In MHC-humanized (heterozygous) ES cells, the MHC region of one allele is humanized. Thus, CRISPR/Cas9-mediated genomic sequence deletion induction or gene disruption, which targets sequences within the mouse MHC region, will all occur on the other allele containing the mouse MHC region (Figure 17). Therefore, if the mouse MHC genes is disrupted/deleted in MHC-humanized (heterozygous) ES cells, a mouse MHC genes-deficient allele can be produced. Since MHC-humanized (heterozygous) mice can be produced from MHC-humanized (heterozygous) ES cells, it is possible to produce mouse MHC genes-deficient mice, if mouse MHC genes-deficient MHC-humanized (heterozygous) ES cells are produced.

### < Outline of present example >

In the present example, mouse MHC genes-deficient alleles were produced using MHC-humanized (heterozygous) ES cells, in which the MHC region from Daxx to Mog was humanized in XO ES cells derived from CBAxC57BL/6 F1 mice. First, looking at distribution of the MHC genes on the genome, it was found that 35 of the 39 genes were present in five clusters without any non-MHC genes (Figure 18). These clusters are designated d1, d2, d3, d4, and d5, in descending order of size. Five genes, H2-K1, H2-Oa, H2-M5, H2-M3, and H2-M2, were found to exist independently without forming a cluster. These isolated MHC genes were classified into d6. The MHC genes present within the cluster were deleted using two CRISPR/Cas9 designed to sandwich the cluster, whereas the isolated MHC genes were disrupted using CRISPR/Cas9 designed to disrupt the protein-coding sequence of each MHC gene.

Specifically, as shown in Figure 19, the MHC genes were sequentially deleted, so that d1 to d6 clones were isolated. The d1 clone is a clone in which the MHC genes in the d1 cluster of MHC-humanized (heterozygous) ES cells have been deleted. The d2 clone is a clone in which the MHC genes in the d2 cluster have been deleted from the d1 clone. The d3 clone is a clone in which the MHC genes in the d3 cluster have been deleted from the d2 clone. The d4 clone is a clone in which the MHC genes in the d4 cluster have been deleted from the d3 clone. The d5 clone is a clone in which the MHC genes in the d5 cluster have been deleted from the d4 clone, and further, the H2-M5 gene has been simultaneously disrupted. The d6 clone is a clone in which the H2-K1, H2-Oa, H2-M3, and H2-M2 genes of the d5 clone are disrupted. Since simultaneous disruption of all four genes reduces gene disruption efficiency in production of the d6 clone, in some cases, the d6a clone was isolated by disrupting only the H2-K1 and H2-M3 genes of the d5 clone, and then, the H2-Oa and H2-M2 genes were disrupted to isolate the d6 clone.

### < Methods >

### Generation of genome editing vectors

For the expression of gRNA and Cas9, there was used PX458.1a, in which pSpCas9(BB)-2A-GFP (PX458) (Addgene plasmid #48138; http://n2t.net/addgene:48138; RRID: Addgene 48138) was modified and the marker fluorescent gene was substituted with ametrine.

For deletion of the d1 cluster, the following vectors were used. The sequences of gRNAs are as follows.
PX458.1a-M10.2upCR1: ATGCTGTACTGCCATACGAA (SEQ ID No: 53)
PX458.1a-M10.6downCRl: GATGTATCCACTCACGCACT (SEQ ID No: 54)

For deletion of the d2 cluster, the following vectors were used. The sequences of gRNAs are as follows.
PX458.1a-H2D1upCR1: ATTAAGGACACTAATACCTG (SEQ ID No: 55)
PX458.la-Q10downCR1: CACACATTGCTGATCTGCTG (SEQ ID No: 56)

For deletion of the d3 cluster, the following vectors were used. The sequences of gRNAs are as follows.
PX458.1a-T24upCR1: AATATGATTGGATTGTACGT (SEQ ID No: 57)
PX458.1a-T3downCR1: CATCAATGCATAGATGTCTG (SEQ ID No: 58)

For deletion of the d4 cluster, the following vectors were used. The sequences of gRNAs are as follows.
PX458.1a-H2ObupCR1: TCATCTGGGGAAGTGCATCG (SEQ ID No: 59)
PX458.1a-H2Eb2CR1: AAGACTGGAGTTGTGTCCAC (SEQ ID No: 60)

For deletion of the d5 cluster and disruption of H2-M5, the following vector were used. The sequences of gRNAs are as follows.
PX458.1a-H2DMaupCR1: GATGCTCTGTTGAAACGTGA (SEQ ID No: 61)
PX458.1a-DMbldownCR1: CCAAGCAATCTGACCTGCTG (SEQ ID No: 62)
PX458.1a-H2-MSCR1: CATGAGAAGCACGCATACGA (SEQ ID No: 63)

For disruption of H2-K1, H2-Oa, H2-M3, and H2-M2 contained in d6, the following vector were used. The sequences of gRNAs are as follows.
PX458.1a-H2M2CR1: CTGAGATCACCCGTAGAGAG (SEQ ID No: 64)
PX458.1a-H2M3CR1: CTTCATCCTTCTGCCAGGTC (SEQ ID No: 65)
PX458.1a-H2OaCR1: GCTGACAGCTCTGGTTCGGT (SEQ ID No: 66)
PX458.1a-H2K1CR1: TCTGCTGTGATGGGTCACAT (SEQ ID No: 67)

### Isolation of d1 clones

For production of d1 clones lacking the mouse MHC genes contained in d1, MHC-humanized (heterozygous) ES cells were transfected with PX458.1a-M10.2upCR1 and PX458.1a-M10.6downCR1, using Lipofectamine 3000 (Thermo Fisher Scientific). The following day, vector-transfected cells were sorted using the expression of the fluorescent gene ametrine as an indicator. Two or three days after the sorting, the cells were re-seeded for clonal isolation. Genomic DNA was prepared from the isolated clones, and clones, from which the d1 clusters were deleted, were identified by PCR. The primers used in PCR are as follows.
M10.2up cel fw1:TCTGATGGAGGTCAGCCAGAC (SEQ ID No: 68)
M10.6down cel rv1:TGTGTTTCCTACTGCAAGGATC (SEQ ID No: 69)

Among the PCR-positive clones, clones with normal chromosome counts were subjected to FISH analysis, which confirmed the deletion of the d1 clusters. In the FISH analysis, Cy3-labeled RP23-147G23 was used to detect the mouse chromosome 17, DY490-labeled RP23-355M14 was used to detect the d1 cluster, and Cy5-labeled RP11-54H13 was used to detect the human MHC region.

### Isolation of d2 clones

For production of d2 clones lacking the mouse MHC genes contained in d1 and d2, the d1 clones were transfected with PX458.1a-H2D1upCR1 and PX458.1a-Q10downCR1, using Lipofectamine 3000. The following day, vector-transfected cells were sorted using the expression of the fluorescent gene ametrine as an indicator. Two or three days after the sorting, the cells were re-seeded for clonal isolation. Genomic DNA was prepared from the isolated clones, and clones, from which the d2 clusters were deleted, were identified by PCR. The primers used in PCR are as follows.
H2D1up cel fw1:TGACGCCATGCTTCGGCTAGC (SEQ ID No: 70)
Q10down cel rv1:CAGACCCAGTTTGAGTCAGTAG (SEQ ID No: 71)

Among the PCR-positive clones, clones with normal chromosome counts were subjected to FISH analysis, which confirmed the deletion of the d2 clusters. In the FISH analysis, Cy3-labeled RP23-147G23 was used to detect the mouse chromosome 17, DY490-labeled RP23-101J16 was used to detect the d2 cluster, and Cy5-labeled RP11-54H13 was used to detect the human MHC region.

### Isolation of d3 clones

For production of d3 clones lacking the mouse MHC genes contained in d1, d2, and d3, the d2 clones were transfected with PX458.1a-T24upCR1 and PX458.1aT3downCR1, using Lipofectamine 3000. The following day, vector-transfected cells were sorted using the expression of the fluorescent gene ametrine as an indicator. Two or three days after the sorting, the cells were re-seeded for clonal isolation. Genomic DNA was prepared from the isolated clones, and clones, from which the d3 clusters were deleted, were identified by PCR. The primers used in PCR are as follows.
T24up cel seq fw1:AAGAGCTTAAGGCTGCTCTG (SEQ ID No: 72)
T3down cel rv1:CCAGGAGATGTCTCAATGCT (SEQ ID No: 73)

Among the PCR-positive clones, clones with normal chromosome counts were subjected to FISH analysis, which confirmed the deletion of the d3 clusters. In the FISH analysis, Cy3-labeled RP23-147G23 was used to detect the mouse chromosome 17, DY490-labeled RP23-268P19 was used to detect the d3 cluster, and Cy5-labeled RP11-54H13 was used to detect the human MHC region.

### Isolation of d4 clones

For production of d4 clones lacking the mouse MHC genes contained in d1, d2, d3, and d4, the d3 clones were transfected with PX458.1a-H2ObupCR1 and PX458.1a-H2Eb2CR1, using Lipofectamine 3000. The following day, vector-transfected cells were sorted using the expression of the fluorescent gene ametrine as an indicator. Two or three days after the sorting, the cells were re-seeded for clonal isolation. Genomic DNA was prepared from the isolated clones, and clones, from which the d4 clusters were deleted, were identified by PCR. The primers used in PCR are as follows.
H2-Ob up cel fw1:TCATCACATAATGTAAAGATACCTGTG (SEQ ID No: 74)
H2-Eb2 CR1 cel rv1:GTGGAGTCACCCATGTGCTT (SEQ ID No: 75)

After confirming that the PCR-positive clones had normal chromosome counts, the clones were used to generate d5 clones.

### Isolation of d5 clones

For production of d5 clones, in which the mouse MHC genes contained in d1, d2, d3, d4, and d5 were deleted, and H2-M5 was disrupted, the d4 clones were transfected with PX458.1a-H2DMaupCR1, PX458.1a-DMb1downCR1 and PX458.1a-H2-M5CR1, using Lipofectamine 3000. The following day, vector-transfected cells were sorted using the expression of the fluorescent gene ametrine as an indicator. Two or three days after the sorting, the cells were re-seeded for clonal isolation. Genomic DNA was prepared from the isolated clones, and clones, from which the d5 clusters were deleted, were identified by PCR. The primers used in PCR are as follows.
H2DMa up cel fw1:ATTTGTAGCCTCACATTCAATGC (SEQ ID No: 76)
DMb1 down cel rv1:GGCTGTTGACTTCCCAACTCTAATC (SEQ ID No: 77)

PCR-positive clones were further analyzed in terms of gene disruption of H2-M5. First, using the genomic DNA of each clone as a template, a sequence containing the target site of PX458.1a-H2-M5CR1 was amplified using the following primers.
H2M5 CR1 cel fw2: TGCTCACCGGAGCTTTGGCCTTGAC (SEQ ID No: 78)
H2M5CR1 cel rv2: ACTGAAGCAATGTTTATGGACCATC (SEQ ID No: 79)

The PCR product was purified using Sephadex G-50 (Cytiva) or FavorPrep GEL/PCR Purification Mini Kit (Favorgen), and whether the target gene was disrupted was confirmed by performing sequence analysis using the following primer. H2M5CR1 cel seq rv2: CAAGATGGACTTCGGGACTC (SEQ ID No: 80)

After confirming that clones, in which deletion of the d5 clusters and disruption of H2-M5 were confirmed, had normal chromosome counts, the clones were used to generate d6 clones.

### Isolation of d6 clones

For production of d6 clones, in which all the mouse MHC genes contained in d1 to d6 were deleted or disrupted, the d5 clones were transfected with PX458.1aH2M2CR1, PX458.1a-H2M3CR1, PX458.1a-H2OaCR1 and PX458.1a-H2K1CR1, using Lipofectamine 3000. The following day, vector-transfected cells were sorted using the expression of the fluorescent gene ametrine as an indicator. Two or three days after the sorting, the cells were re-seeded for clonal isolation. If all the above-described four genes are simultaneously disrupted, the efficiency capable of isolating the d6 clones becomes low. Thus, in some cases, d6a clones, in which the H2-K1 and H2-M3 of the d5 clones were disrupted, were isolated, and then, H2-Oa and H2-M2 were disrupted to generate d6 clones.

Genomic DNA was prepared from the isolated clones, and a sequence containing the target site of gRNA designed with respect to each MHC gene was amplified using the following primers.
H2K1
   H2-K1 CR1 cel fw1:CCTTCCAGGATCATACAGCC (SEQ ID No: 81)
   H2-K1 CR1 cel rv1:CTCCACAAGCTCCATGTCCT (SEQ ID No: 82)
H2-M3
   H2-M3 CR1 cel fw1: TGGATCCAGACTCCCGTATG (SEQ ID No: 83)
   H2-M3 CR1 cel rv1: GTGAGAGTCATGATGCCCAG (SEQ ID No: 84)
H2-M2
   H2-M2 CR1 cel fw1: GGAGGATGAAAGGGCTAAGC (SEQ ID No: 85)
   H2-M2 CR cel rv1: CTCAGGAGACATGAGCTCCAG (SEQ ID No: 86)
H2-Oa
   H2-Oa CR1 cel fw1: TTTGGAGACTTCGCCCACTC (SEQ ID No: 87)
   H2-Oa CR1 cel rv1: ACTCTGGGAGGCACTAGGAG (SEQ ID No: 88)

Each PCR product was purified using Sephadex G-50 or FavorPrep GEL/PCR Purification Mini Kit, and whether the target gene was disrupted was confirmed by performing sequence analysis using the following primers.
H2K1
   H2-K1 CR1 seq fw1: CAATGGCTCATGTGGATTCC (SEQ ID No: 89)
H2-M3
   H2-M3 CR1 seq rv1: CTTCCTTACCCCATTTCAGG (SEQ ID No: 90)
H2-M2
   H2-M2 CR seq fw1: CTACTTCACGCATACCGTCAG (SEQ ID No: 91)
H2-Oa
   H2-Oa CR1 seq rv1: CACCTGCAGAAACGTTCTGG (SEQ ID No: 92)

In SNP analysis upstream and downstream of the gRNA target site of H2-M2, the genomic sequence containing the SNP site was amplified, and was then confirmed by sequence analysis. Each PCR product was purified using Sephadex G-50 or the FavorPrep GEL/PCR Purification Mini Kit. The primer sequences used in the amplification of the genomic sequence and the sequence analysis are as follows.

Amplification of genomic sequence containing SNP site (GRCm38/mm10, chr17:37466727) located upstream of H2-M2 gRNA target site
H2M2 up SNP fw1: ATCATTCCTAGGCTGCATCACTCAG (SEQ ID No: 93)
H2M2 up SNP rv1: CCTCACTCATGGAGGAGGCTGATGC (SEQ ID No: 94)
Sequence analysis
H2M2 up SNP seq fw1: TTCACTTTCTGGGAAGTACG (SEQ ID No: 95)

The present SNP sequence is A in CBA, and C in C57BL/6.

Amplification of genomic sequence containing SNP site (GRCm38/mm10, chr17: 37491000, 37491007) located downstream of H2-M2 gRNA target site
H2-M2 down SNP fw1: GACCGTAGGACCATTTGCCATGTTC (SEQ ID No: 96)
H2-M2 down SNP rv1: TCACTGATACCAAGCAGTAGTTCTG (SEQ ID No: 97)
Sequence analysis
H2-M2 down SNP seq fw1: ACACTTGAAGTTATTGTCATACAG (SEQ ID No: 98)

Regarding the SNP sequence, 37491000 is C in CBA and T in C57BL/6, whereas 37491007 is C in CBA and A in C57BL/6.

It was confirmed that clones, in which gene disruption and the maintenance of SNP were confirmed, had normal chromosome counts.

### Analysis of d5 homozygous mice

In the analysis of CD4-positive T cells, splenocytes or thymocytes were prepared from wild-type or d5 homozygous mice, and were stained with anti-CD3, CD4, and CD8 antibodies (Biolegend). The percentages of CD4- and CD8-positive cells in CD3-positive cells were analyzed using a FACSAriaIII (BD Biosciences).

In class switching analysis, 11- to 15-week-old wild-type mice (n = 3) or d5 homozygous mouse (n = 1) were subcutaneously immunized with 10 µg of recombinant SARS-CoV-2 S Protein S1+S2 (Biolegend) and 50 µg of poly(I:C) (InvivoGen). Three weeks later, they were further boosted with 10 µg of recombinant SARS-CoV-2 S Protein S1+S2 and 50 µg of poly(I:C). One week after the booster immunization, serum was collected, and was analyzed for anti-S1 IgG1 and IgG2 by ELISA.

### Analysis of d6 chimeric mice

In the analysis of MHC genes-deficient chromosomes, deletion of the d5 cluster was identified using the following primers. The size of the PCR product was 447 bp in wild-type mice, and was 633 bp in the d5 deletion in the case of the ES clones used to produce d6 mice.
Fw1: ATTTGTAGCCTCACATTCAATGC (SEQ ID No: 99)
Rv1: GGCTGTTGACTTCCCAACTCTAATC (SEQ ID No: 100)
Rv2: AGCACAGCTCCTGACTTCTG (SEQ ID No: 101)

### < Results >

### Isolation of d1 clones

gRNAs were designed to a portion upstream of the H2-M10.2 locus and within the protein-coding sequence of the H2-M10.6 locus. The d1 cluster in MHC-humanized (heterozygous) ES cells was induced to be deleted according to CRISPR/Cas9 using these two gRNAs, and d1 clones were isolated (Figure 20). Genomic PCR was carried out using a primer set designed to generate a PCR amplified product when the genomic sequence flanked by the two CRISPR/Cas9 target sequences was deleted. As a result, multiple PCR-positive clones were obtained. Among the PCR-positive clones, d1 clone #26 could be confirmed to have a normal chromosome count (39 in XO ES cells). The d1 clone #26 was subjected to FISH analysis using a probe that recognizes the d1 cluster, and as a result, deletion of the d1 cluster was confirmed.

### Isolation of d2 clones

gRNAs were designed to a portion upstream of the H2-D1 locus and within the protein-coding sequence of the H2-Q10 locus. The d2 cluster in the d1 clone #26 was induced to be deleted according to CRISPR/Cas9 using these two gRNAs, and d2 clones were isolated (Figure 21). Genomic PCR was carried out using a primer set designed to generate a PCR amplified product when the genomic sequence flanked by the two CRISPR/Cas9 target sequences was deleted. As a result, multiple PCR-positive clones were obtained. Among the PCR-positive clones, d2 clone #85 could be confirmed to have a normal chromosome count. The d2 clone #85 was subjected to FISH analysis using a probe that recognizes the d2 cluster, and as a result, deletion of the d2 cluster was confirmed.

### Isolation of d3 clones

gRNAs were designed to a portion upstream of the H2-T24 locus and a portion downstream of the H2-T3 locus. The d3 cluster in the d2 clone #85 was induced to be deleted according to CRISPR/Cas9 using these two gRNAs, and d3 clones were isolated (Figure 22). Genomic PCR was carried out using a primer set designed to generate a PCR amplified product when the genomic sequence flanked by the two CRISPR/Cas9 target sequences was deleted. As a result, multiple PCR-positive clones were obtained. Among the PCR-positive clones, d3 clone #26 could be confirmed to have a normal chromosome count. The d3 clone #26 was subjected to FISH analysis using a probe that recognizes the d3 cluster, and as a result, deletion of the d3 cluster was confirmed.

### Isolation of d4 clones

gRNAs were designed to a portion upstream of the H2-Ob locus and within the protein-coding sequence of the H2-Eb2 locus. The d4 cluster in the d3 clone #26 was induced to be deleted according to CRISPR/Cas9 using these two gRNAs, and d4 clones were isolated (Figure 23). Genomic PCR was carried out using a primer set designed to generate a PCR amplified product when the genomic sequence flanked by the two CRISPR/Cas9 target sequences was deleted. As a result, multiple PCR-positive clones were obtained. Further, it was confirmed that PCR-positive d4 clone #72 had a normal chromosome count.

### Isolation of d5 clones

gRNAs were designed within the protein-coding sequence of the H2-DMa locus and a portion downstream of the H2-DMbl locus. The d5 cluster in the d4 clone #72 was induced to be deleted according to CRISPR/Cas9 using these two gRNAs. Simultaneously, using the gRNA designed within the protein-coding sequence of the H2-M5 locus, the H2-M5 gene was disrupted, and d5 clones were isolated (Figure 24). Genomic PCR was carried out using a primer set designed to generate a PCR amplified product when the genomic sequence flanked by the above-described two CRISPR/Cas9 target sequences was deleted. As a result, multiple PCR-positive clones were obtained. Subsequently, the PCR-positive clones were subjected to sequence analysis to examine whether the H2-M5 gene had been disrupted. As a result, deletion of 13 nucleotides was confirmed in d5 clone #22. Further, chromosome count analysis was carried out on clones in which disruption of the H2-M5 gene had been confirmed, and as a result, it could be confirmed that the d5 clone #22 had a normal chromosome count.

### Isolation of d6 clones

Using gRNAs designed within the protein-coding sequences of each of H2-K1, H2-Oa, H2-M2 and H2-M3, gene disruption was performed on d5 clone #22 according to CRISPR/Cas9, and d6 clones were isolated (Figure 25).

To confirm whether or not each gene was disrupted by CRISPR/Cas9, sequence analysis was performed and clones in which all four genes were disrupted were identified. Since H2-M2 and H2-M3 are located outside the MHC-humanized region, two copies of the gene exist in the genome. Since the mutations occur separately in each allele, it is likely that two types sequences may be detected by sequence analysis. The ES cells used in the present example were derived from an F1 cross between CBA and C57BL/6, and the human MHC region is present in the CBA-derived chromosome. Thus, regarding H2-M3, gene disruption was confirmed using sequence primers capable of specifically detecting the C57BL/6 allele utilizing SNP. Regarding H2-M2, since there were no SNPs near the CRISPR/Cas9 target site, allele-specific sequence analysis was difficult. Thus, sequence analysis was carried out using primers that detect both alleles. As a result, only one type of sequence was detected in d6 clone #198 shown in Figure 25. This may be because both alleles contain the same mutation, or because introduction of multiple CRISPR/Cas9 induced a large deletion between CRISPR/Cas9 target sequences adjacent, so that only one allele that could be amplified by PCR may remain.

Thus, whether SNPs near H2-M2 could be detected was analyzed, and whether a large deletion had occurred was confirmed (Figure 26). As a result, in clone #198, SNPs were detected upstream and downstream of H2-M2, and thus, it was confirmed that no large deletion had been induced. Since #198 also had a normal chromosome count, it is considered that ES cells having alleles in which all 39 types of mouse MHC genes were disrupted were successfully produced.

### Production of d5 clone-derived mice

In the d5 ES clones isolated in the process of deleting mouse MHC, 35 of the 39 types of mouse MHC genes were disrupted or deleted, and thus, these clones do not have mouse MHC class II, which functions in antigen presentation. Hence, d5 homozygous mice were produced using the d5 ES clone, and whether the function of the mouse MHC class II function had actually been lost was analyzed.

First, differentiation of T cells in wild-type and d5 homozygous mice was analyzed. Since antigen presentation by the MHC class II is not possible in the d5 homozygous mice, it is considered that CD4-positive T cells do not come to differentiate therein. Thus, the proportion of CD4-positive T cells in the spleen and thymus of the d5 homozygous mice was analyzed, and as a result, it was found that the proportion thereof was significantly reduced (Figure 27). Moreover, to analyze antibody class switching in the d5 homozygous mice, the d5 homozygous mice were immunized with the spike protein of SARS-CoV-2, and anti-S1 IgG1 and IgG2 in the serum were analyzed. As a result, it was found that IgG1 and IgG2 recognizing the spike protein were extremely small or absent in the d5 homozygous mice. From these results, it is considered that functional CD4-positive T cells are significantly reduced or disappear in the d5 homozygous mice because mouse MHC class II, which functions in antigen presentation, is not expressed therein.

### Production of mouse MHC genes-deficient mice

To establish mice with disrupted or deleted all mouse MHC genes, chimeric mice were produced from the d6 ES clones and germline transmission was used to produce d6 heterozygous mice (Figures 28A and B). Subsequently, these d6 heterozygous mice were bred with each other, and the resulting offsprings were analyzed by genomic PCR. As a result, mouse MHC genes-deficient mice, homozygously having MHC-deficient chromosomes, were confirmed (Figures 28C and D).

## Claims

1. A chromosome of a non-human mammal, in which all or a part of the major histocompatibility complex (MHC) genes is deleted or disrupted.

2. The chromosome according to claim 1, wherein the non-human mammal is a rodent.

3. The chromosome according to claim 2, wherein the rodent is a mouse.

4. A cell that homozygously or heterozygously comprises the chromosome according to claim 1.

5. The cell according to claim 4, which is an embryonic stem cell.

6. A non-human mammal having the chromosome according to claim 1.

7. A method for producing a cell that has a chromosome having the MHC genes (MHC genes 2) of a mammal 2 in the chromosome of a non-human mammal 1, and a chromosome in which all or a part of an MHC genes 1 is disrupted or deleted, wherein the production method comprises a step of disrupting or deleting all or a part of the MHC genes (MHC genes 1) derived from one non-human mammal (non-human mammal 1), from a cell heterozygously having a chromosome in which all or a part of the major histocompatibility complex (MHC) genes of one wild-type chromosome, between a pair of chromosomes of the non-human mammal, is substituted with the MHC genes of another mammal (mammal 2) other than the non-human mammal 1.

8. The method according to claim 7, wherein the cell is an embryonic stem cell.

9. The method according to claim 7, wherein the step of disrupting or deleting the MHC genes 1 is carried out by genome editing technology using CRISPR/Cas9.

10. The method according to claim 7, wherein the non-human mammal 1 is a rodent.

11. The method according to claim 10, wherein the rodent is a mouse.

12. The method according to claim 11, wherein the step of disrupting or deleting all or a part of the MHC genes 1 includes a step of disrupting or deleting, by genome editing technology using CRISPR/Cas9, all or a part of genes consisting of a d1 genes, a d2 genes, a d3 genes, a d4 genes, a d5 genes and a d6 genes as described below, from a cell having a mouse MHC genes that consists of the d1 genes consisting of the genes H2-M10.2, H2-M10.1, H2-M10.3, H2-M10.4, H2-M11, H2-M9, H2-M1, H2-M10.5 and H2-M10.6, the d2 genes consisting of the genes H2-D1, H2-Q1, H2-Q2, H2-Q4, H2-Q5, H2-Q6, H2-Q7 and H2-Q10, the d3 genes consisting of the genes H2-T24, H2-T23, H2-T22, GM11127, H2-BI, H2-T10, GM7030, GM8909 and H2-T3, the d4 genes consisting of the genes H2-Ob, H2-Ab1, H2-Aa, H2-Eb1 and H2-Eb2, the d5 genes consisting of the genes H2-DMa, H2-DMb2 and H2-DMb1, and the d6 genes consisting of the genes H2-K1, H2-Oa, H2-M5, H2-M3 and H2-M2.

13. The method according to claim 12, wherein the genome editing vector used in the genome editing technology using CRISPR/Cas9 is a combination of vectors consisting of gRNAs having the nucleotide sequences as set forth in SED ID NOs: 53 to 67.

14. A method for producing a non-human mammal 1 having a chromosome in which all or a part of the MHC genes 1 is disrupted or deleted, wherein the production method comprises a step of using the cell obtained by the method according to claim 7 to produce a non-human mammal 1 having a chromosome having the MHC genes 2 of the mammal 2 and a chromosome in which all or a part of the MHC genes 1 is disrupted or deleted, in a pair of chromosomes of the non-human mammal 1, and then using the non-human mammal 1 for breeding.

15. A method for producing a cell having a chromosome in which all or a part of the MHC genes 1 is disrupted or deleted, wherein the production method comprises a step of recovering the cell from the non-human mammal 1 produced by the method according to claim 14.

16. A method for producing a chromosome in which the MHC genes 1 is disrupted or deleted, wherein the production method comprises a step or recovering the chromosome from the non-human mammal 1 produced by the method according to claim 14, or the cell produced by the method according to claim 15.

17. The genome editing vector used in the genome editing technology using CRISPR/Cas9 according to claim 13, wherein the genome editing vector includes a combination of vectors consisting of gRNAs having the nucleotide sequences as set forth in SED ID NOs: 53 to 67.
